# EUROPÄISCHE PATENTSCHRIFT

(11) **EP 0 542 059 B1**
(45) Veröffentlichungstag und Bekanntmachung des Hinweises auf die Patenterteilung: **20.03.1996**
(21) Anmeldenummer: 92118597.1
(22) Anmeldetag: 30.10.1992
(51) Int. Cl.: C07D 401/10, C07D 401/14, C07D 213/79, A61K 31/44

(54) **Substituierte Biphenylpyridone als Angiotensin II Antagonisten**
Substituted biphenylpyridinones as angiotensin II antagonists
Biphénylpyridinones substitués comme antagonistes de l'angiotensine II

(30) Priorität: 12.11.1991 DE 4137151; 01.07.1992 DE 4221583
(43) Veröffentlichungstag der Anmeldung: 19.05.1993
(73) Patentinhaber: BAYER AG, D-51368 Leverkusen (DE)
(72) Erfinder: Müller-Gliemann, Matthias, Dr., W-5650 Solingen-Ohligs (DE); Beuck, Martin, Dr., W-4006 Erkrath 2 (DE); Kazda, Stanislav, Prof. Dr., W-5600 Wuppertal 1 (DE); Stasch, Johannes-Peter, Dr., W-5600 Wuppertal 1 (DE); Knorr, Andreas, Dr., W-4006 Erkrath 2 (DE); Wohlfeil, Stefan, Dr., W-4010 Hilden (DE); Hübsch, Walter, Dr., W-5600 Wuppertal 1 (DE); Dressel, Jürgen, Ph.D., W-5600 Wuppertal 1 (DE); Fey, Peter, Dr., W-5600 Wuppertal 1 (DE); Hanko, Rudolf, Dr., W-4300 Essen (DE); Krämer, Thomas, Dr., W-5600 Wuppertal 1 (DE); Müller, Ulrich, Dr., W-5600 Wuppertal 1 (DE); Zaiss, Siegfried, Dr., W-5600 Wuppertal 1 (DE)

(56) Entgegenhaltungen:
- EP-A- 530 702
- EP-A- 0 435 827
- EP-A- 0 445 811
- EP-A- 0 487 745
- EP-A- 0 500 297
- WO-A-92/14714

## Beschreibung

Die Erfindung betrifft substituierte Biphenylpyridone, Verfahren zu ihrer Herstellung sowie ihre Verwendung in Arzneimitteln, insbesondere als blutdrucksenkendes und anti-atherosklerotisches Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, Na^{*-} Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind aus den Publikationen EP 407 342, 424 317,435 827 und 419 048 Biphenyl substituierte Pyrimidone bekannt.

In der EP-A 445 811 werden biphenylsubstituierte Nicotinsäure-Derivate mit Angiotensin II autagonistischen Eigenschaften offenbart.

Die EP-A 500 297 sowie EP-A 530 702 betreffen substituierte Biphenylpyridone zur Behandlung von Bluthochdruck und anderen, durch Angiotensin-II-Antagonisten beeinflußbare Krankheiten. Bei beiden Dokumenten handelt es sich uni Stand der Technik gemäß Regel 54(3) EPÜ.

Die Erfindung betrifft substituierte Biphenylpyridone der allgemeinen Formel (I) 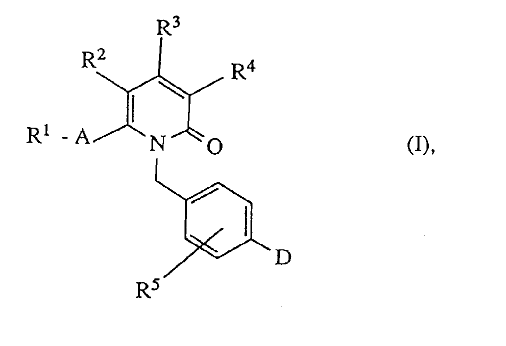 in welcher
R¹ für geradkettiges oder verzweigtes Alkyl mit bis zu 10 Kohlenstoffatomen steht, das gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen substituiert ist, oder für Cycloalkyl mit 3 bis 6 Kohlenstoffatomen oder Halogen steht,
A für ein Sauerstoff- oder Schwefelatom steht oder für die Gruppe der Formel -NR⁶ steht worin
R⁶ Wasserstoff oder geradkettiges oder verzweigtes Alkyl mit bis zu 6 Kohlenstoffatomen bedeutet,
   oder
R6 gemeinsam mit R¹ unter Einbezug des Stickstoffatoms einen 5-oder 6-gliedrigen, gesättigten oder ungesättigten Heterocyclus bildet,
R2, R3 und R4 gleich oder verschieden sind und
   für Wasserstoff, Nitro, Cyano, Formyl oder Halogen stehen, oder für geradkettiges oder verzweigtes Alkyl, Alkenyl, Alkinyl, Alkoxy oder Alkylthio mit jeweils bis zu 8 Kohlenstoffatomen stehen, die gegebenenfalls bis zu 2-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Benzyl, Phenyl, Phenoxy, Benzoyl oder durch einen 5- bis 7-gliedrigen, gesättigten oder ungesättigten Heterocyclus mit bis zu 3 Heteroatomen substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
   für geradkettiges oder verzweigtes Acyl oder Alkoxycarbonyl mit jeweils bis zu 8 Kohlenstoffatomen, Phenoxycarbonyl, Benzyloxycarbonyl oder Carboxy stehen, oder
   für Cycloalkyl oder -alkenyl mit 3 bis 8 Kohlenstoffatomen, für einen 5- bis 7-gliedrigem, ungesättigtem Heterocyclus mit bis zu 3 Heteroatomen aus der Reihe S, N oder O, Phenyl, Phenoxy oder Phenylthio stehen, die gegebenenfalls bis zu 3-fach gleich oder verschieden durch Halogen, Nitro, Cyano, Hydroxy, Hydroxymethyl, Trifluormethyl, Trifluormethoxy oderdurch geradkettiges oderverzweigtes Alkyl oder Alkoxy mit jeweils bis zu 6 Kohlenstoffatomen substituiert sind, oder,
   für Tetrazolyl stehen, die gegebenenfalls durch Methyl oder eine Tritylgruppe substituiert sind oder
   für eine Gruppe der Formel 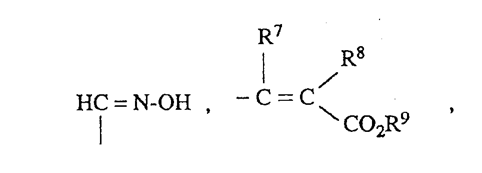 -NR¹⁰R¹¹, -CO-NR¹⁰R¹¹ oder -CH2-OR12 stehen
   worin
R⁷ Wasserstoff oder geradkettiges oder verzweigtes Alkyl mit bis zu 6 Kohlenstoffatomen bedeutet,
R8 Wasserstoff oder geradkettiges oder verzweigtes Alkyl mit bis zu 8 Kohlenstoffatomen bedeutet, das gegebenenfalls durch Cycloalkyl mit 3 bis 6 Kohlenstoffatomen, Phenyl oder durch einen 5- bis 7- gliedrigen gesättigten oder ungesättigten Heterocyclus mit bis zu 3 Heteroatomen aus der Reihe S, N oder O substituiert ist, wobei die Cyclen gegebenenfalls durch Hydroxy, Hydroxymethyl, Halogen oder durch geradkettiges oder verzweigtes Alkyl oder Alkoxy mit jeweils bis zu 6 Kohlenstoffatomen substituiert sind, oder Cycloalkyl mit 3 bis 6 Kohlenstoffatomen oder Phenyl bedeutet,
R9 Wasserstoff, geradkettiges oder verzweigtes Alkyl mit bis zu 8 Kohlenstoffatomen oder Phenyl bedeutet,
R¹ und R¹¹ gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
R12 geradkettiges oder verzweigtes Acyl mit bis zu 6 Kohlenstoffatomen oder Benzoyl bedeutet,
oder -A-R¹ und R² gemeinsam für eine Alkylenkette mit bis zu 5 Kohlenstoffatomen stehen,
R⁵ für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen steht,
D für einen Rest der Formel 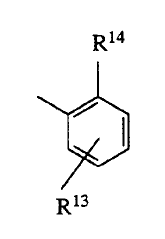 steht,
   worin
R¹3 3 die oben angegebene Bedeutung von R⁵ hat und mit dieser gleich oder verschieden ist,
   und
R¹⁴ eine Gruppe der Formel -CO-R¹⁵, -CO-NR¹⁶R¹⁷ oder -S0₂R18 bedeutet, worin
R¹⁵ Hydroxy oder geradkettiges oder verzweigtes Alkoxy mit bis zu 6 Kohlenstoffatomen bedeutet,
R¹⁶ und R¹⁷ gleich oder verschieden sind und die oben angegebene Bedeutung von R¹⁰ und R¹¹ haben, oder
R¹⁶ Wasserstoff bedeutet und
R¹⁷ die Gruppe -S0₂R¹⁸ bedeutet,
R¹⁸ Hydroxy, geradkettiges oder verzweigtes Alkoxy oder Alkyl mit jeweils bis zu 6 Kohlenstoffatomen, Amino oder (C₁-C₆)-Mono- oder -dialkylamino oder Phenyl bedeutet, das gegebenenfalls bis zu 2-fach gleich oder verschieden durch Halogen, Trifluormethyl oder geradkettiges oder verzweigtes Alkyl mit bis zu 4 Kohlenstoffatomen substituiert sein kann,
   oder
R¹⁴ einen Rest der Formel 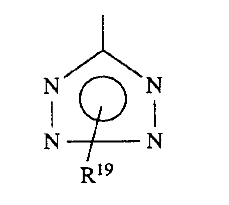 bedeutet,
   worin
R¹9 9 Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,

und deren Salze.

Die erfindungsgemäßen substituierten Biphenylpyridone können auch in Form ihrer Salzevorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Bevorzugt sind Verbindungen der allgemeinen Formel (I),
in welcher
R¹ für geradkettiges oder verzweigtes Alkyl mit jeweils bis zu 8 Kohlenstoffatomen steht, das gegebenenfallsdurch Cyclopropyl, Cyclopentyl, Cyclohexyl, Hydroxy oder durch geradkettiges oder verzweigtes Alkoxy mit bis zu 4 Kohlenstoffatomen substituiert ist, oder für Cyclopropyl, Cyclopentyl, Cyclohexyl, Fluor, Chlor, Brom oder lod steht,
A für ein Sauerstoff- oder Schwefelatom oder die NH-Gruppe steht,
R2, R3 und R4 gleich oder verschieden sind und
   für Wasserstoff, Nitro, Cyano, Formyl, Fluor, Chlor, Brom oder lod stehen, oder
   für geradkettiges oder verzweigtes Alkyl, Alkenyl, Alkinyl, Alkoxy oder Alkylthio mit jeweils bis zu 6 Kohlenstoffatomen stehen, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, lod oder durch geradkettiges oder verzweigtes Alkyl oder Alkoxy mit jeweils bis zu 6 Kohlenstoffatomen substituiert sein können, oder
   für geradkettiges oder verzweigtes Acyl oder Alkoxycarbonyl mit jeweils bis zu 6 Kohlenstoffatomen, Phenoxycarbonyl, Benzyloxycarbonyl oder Carboxy stehen, oder
   für Cyclopentenyl, Cyclopropyl, Cyclopentyl, Cyclohexyl, Thienyl, Furyl, Phenyl, Phenoxy oder Phenylthio stehen, die gegebenenfalls bis zu 2-fach gleich oder verschieden durch Fluor, Chlor, Brom, lod, Trifluormethyl, Trifluormethoxy, Hydroxymethyl oder durch geradkettiges oder verzweigtes Alkyl oder Alkoxy mit jeweils bis zu 4 Kohlenstoffatomen substituiert sind, oder
   für Tetrazolyl stehen, die gegebenenfalls durch Methyl oder eine Tritylgruppe substituiert sind oder für eine Gruppe der Formel 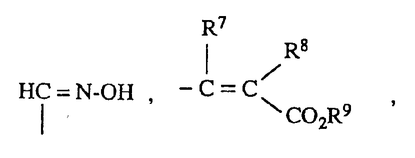 -NR¹⁰R¹¹, -CO-NR¹⁰R¹¹ oder -CH₂-OR¹² stehen,
      worin
R⁷ Wasserstoff oder geradkettiges oder verzweigtes Alkyl mit bis zu 4 Kohlenstoffatomen bedeutet,
R⁸ Wasserstoff oder geradkettiges oder verzweigtes Alkyl mit bis zu 6 Kohlenstoffatomen bedeutet, das gegebenenfalls durch Cyclopropyl, Cyclohexyl, Thienyl oder Phenyl substituiert ist, wobei die Cyclen gegebenenfalls durch Hydroxy, Hydroxymethyl oder geradkettiges oder verzweigtes Alkyl oder Alkoxy mit jeweils bis zu 4 Kohlenstoffatomen substituiert sind,
R9 Wasserstoff, geradkettiges oder verzweigtes Alkyl mit bis zu 6 Kohlenstoffatomen bedeutet,
R¹⁰ und R11 gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
R¹² geradkettiges oder verzweigtes Acyl mit bis zu 6 Kohlenstoffatomen oder Benzoyl bedeutet,
oder -A-R¹ und R² gemeinsam für eine Alkylenkette mit bis zu 5 Kohlenstoffatomen stehen,
R⁵ für Wasserstoff, Fluor, Chlor, Brom, Hydroxy, Trifluormethyl, Trifluormethoxy oder für geradkettiges oder verzweigtes Alkyl oder Alkoxy mit jeweils bis zu 4 Kohlenstoffatomen steht,
D für einen Rest der Formel 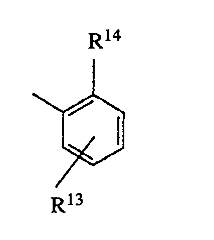 steht,
   worin
R¹3 3 die oben angegebene Bedeutung von R⁵ hat und mit dieser gleich oder verschieden ist
   und
R¹⁴ eine Gruppe der Formel -CO-R¹⁵, -CO-NR¹⁶R¹⁷ oder -SO₂R¹⁸ bedeutet,
   worin
R¹⁵ Hydroxy oder geradkettiges oder verzweigtes Alkoxy mit bis zu 4 Kohlenstoffatomen bedeutet,
R¹6 und R¹⁷ gleich oder verschieden sind und die oben angegebene Bedeutung von R¹⁰ und R¹¹ haben oder
R¹⁶ Wasserstoff bedeutet und
R¹⁷ die Gruppe -SO₂R¹⁸ bedeutet,
R¹⁸ geradkettiges oder verzweigtes Alkyl mit bis zu 4 Kohlenstoffatomen oder p-Tolyl bedeutet,
   oder
R¹⁴ einen Rest der Formel 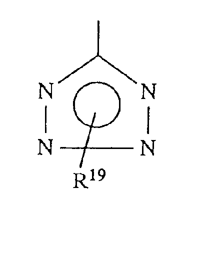 bedeutet,
   worin
R19 9 Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,

und deren Salze.

Besonders bevorzugt sind Verbindungen der allgemeinen Formel (I),
in welcher
R¹ für geradkettiges oder verzweigtes Alkyl mit bis zu 6 Kohlenstoffatomen steht, das gegebenenfalls durch Cyclopropyl, Hydroxy oder Methoxy substituiert ist oder
   für Cyclopropyl, Chlor oder lod steht,
A für ein Sauerstoffatom steht,
R2, R3 und R4 gleich oder verschieden sind und
   für Wasserstoff, Nitro, Cyano, Formyl, Fluor, Chlor, Brom oder lod stehen, oder
   für geradkettiges oder verzweigtes Alkyl, Alkenyl, Alkinyl, Alkoxy oder Alkylthio mit jeweils bis zu 4 Kohlenstoffatomen stehen, die gegebenenfalls durch Hydroxy, Cyano oder durch geradkettiges oder verzweigtes Alkoxy oder Alkoxycarbonyl mit jeweils bis zu 3 Kohlenstoffatomen, Benzyl, Phenyl, Phenoxy oder Benzoyl substituiert sind für geradkettiges oder verzweigtes Acyl oder Alkoxycarbonyl mit jeweils bis zu 4 Kohlenstoffatomen, Phenoxycarbonyl, Benzyloxycarbonyl oder Carboxy stehen, oder
   für Cyclopropyl, Cyclopentyl, Thienyl, Phenyl, Phenoxy oder Phenylthio stehen, die gegebenenfalls durch Fluor, Chlor, Brom, lod oder Trifluormethyl substituiert sind, oder
   für Tetrazolyl stehen, die gegebenenfalls durch Methyl oder eine Tritylgruppe substituiert sind oder für eine Gruppe der Formel 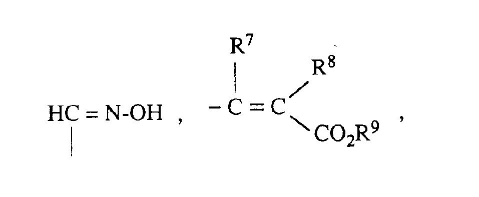 -CO-NR¹⁰R¹¹ oder-CH₂-OR¹² stehen,
      worin
R7 Wasserstoff oder Methyl bedeutet,
R⁸ Wasserstoff oder geradkettiges oder verzweigtes Alkyl mit bis zu 4 Kohlenstoffatomen bedeutet, das gegebenenfalls durch Phenyl substituiert ist, das seinerseits durch Hydroxy, Hydroxymethyl oder durch geradkettiges oder verzweigtes Alkyl oder Alkoxy mitjeweils bis zu 3 Kohlenstoffatomen substituiert ist,
R9 Wasserstoff, geradkettiges oder verzweigtes Alkyl mit bis zu 4 Kohlenstoffatomen bedeutet,
R¹⁰ und R¹¹ gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist,
R12 geradkettiges oder verzweigtes Acyl mit bis zu 4 Kohlenstoffatomen oder Benzoyl bedeutet,
oder -A-R¹ und R² gemeinsam für eine Alkylenkette mit bis zu 4 Kohlenstoffatomen stehen
R5 für Wasserstoff, Fluor, Chlor oder Methyl steht,
D für einen Rest der Formel 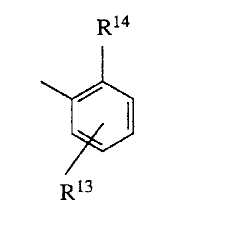 steht,
   worin
R¹³ Wasserstoff bedeutet, und
R¹⁴ eine Gruppe der Formel -CO-R¹⁵, -CO-NR¹6R¹⁷ oder -S0₂-R¹8 bedeutet, worin
R¹⁵ Hydroxy oder geradkettiges oder verzweigtes Alkoxy mit bis zu 3 Kohlenstoffatomen bedeutet,
R¹⁶ und R¹⁷ gleich oder verschieden sind und die oben angegebene Bedeutung von R¹⁰ und R¹¹ haben oder
R¹⁶ Wasserstoff bedeutet und
R¹⁷ die Gruppe -SO₂-R¹⁸ bedeutet,
   worin
R¹⁸ Methyl oder p-Tolyl bedeutet,
   oder
R¹⁴ den Tetrazolylrest der Formel 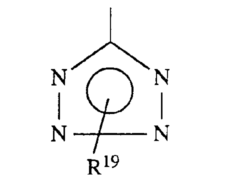 bedeutet
   worin
R¹9 9 Wasserstoff oder geradkettiges oder verzweigtes Alkyl mit bis zu 4 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,

und deren Salze.

Außerdem wurde eine Verfahren zur Herstellung der erfindungsgemäßen Verbindungen der allgemeinen Formel (I) gefunden, dadurch gekennzeichnet, daß man Pyridone der allgemeinen Formel (II) 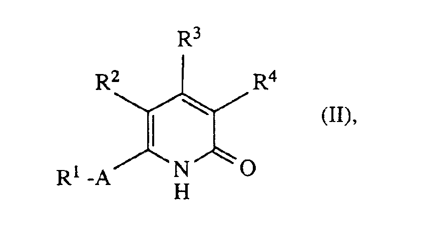 in welcher
R¹, R², R⁴ und A die oben angegebene Bedeutung haben,
mit Verbindungen der allgemeinen Formel (III) 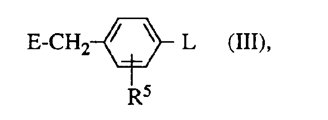 in welcher
E für Halogen, vorzugsweise für Brom steht und
L für einen Rest der Formel 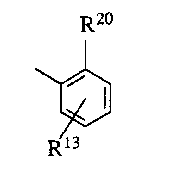 steht,
   worin
R¹ 3 die oben angegebene Bedeutung hat und
R²⁰ C₁-C₄-Alkoxycarbonyl oder
   einen Rest der Formel 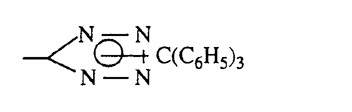 bedeutet,
   in inerten Lösemitteln, in Anwesenheit einer Base und gegebenenfalls unter Zusatz eines Katalysators umsetzt,
   und anschließend im Fall des Tetrazolrestes die Triphenylmethylgruppe mit Säuren in organischen Lösemitteln und/oder Wasser nach üblichen Bedingungen abspaltet,
   und gegebenenfalls im Fall der unter den Substituenten R¹⁴ und/oder R²⁰ aufgeführten carbonylischen Reste nach Verseifung der jeweiligen Ester beispielsweise durch Amidierung oder Sulfoamidierung nach üblichen Methoden derivatisiert,
   und gegebenenfalls auch die Substituenten R², R³, R⁴, R⁵, R¹³ und R¹⁹ nach bekannten Methoden variiert.

Das erfindungsemäße Verfahren kann durch folgendes Formelschema beispielhaft erläutert werden: 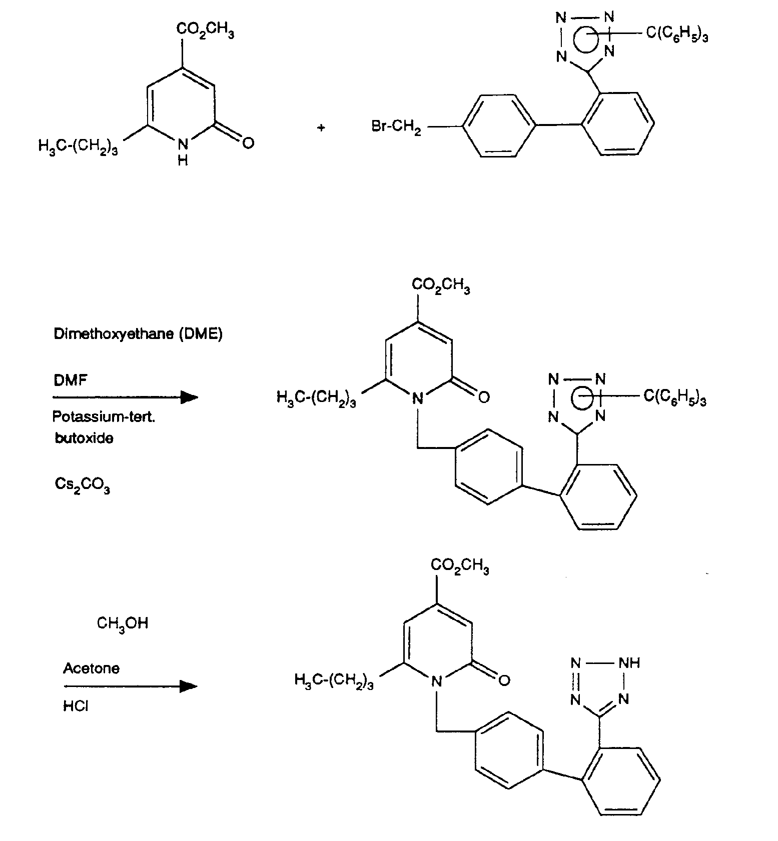 Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel zu verwenden. Bevorzugt ist Tetrahydrofuran, Aceton, Dimethylformamid und Dimethoxyethan.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkali- oder Erdalkalialkoholate oder -amide wie Natrium- oder Kaliummethanolat, Natrium- oder Kaliumethanolat oder Kalium-tert.butylat, oder Lithiumdiisopropylamid (LDA), oder organische Amine (Trialkyl(C₁-Cₑ)-amine) wie Triethylamin, oder Heterocyclen wie 1,4-Diazabi- cyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumhydrid, Kalium-tert.-butylat oder Caesiumcarbonat.

Im allgemeinen setzt man die Base in einer Menge von 0,05 mol bis 10 mol, bevorzugt von 1 mol bis 2 mol bezogen auf 1 mol der Verbindung der Formel (111) ein.

Das erfindungsgemäße Verfahren wird im allgemeinen in einem Temperaturbereich von -100°C bis +100°C, bevorzugt von 0°C bis 80°C durchgeführt.

Das erfindungsgemäße Verfahren wird im allgemeinen bei Normaldruck durchgeführt. Es ist aber auch möglich, das Verfahren bei ]berdruck oder bei Unterdruck durchzuführen (z.B. in einem Bereich von 0,5 bis 5 bar).

Als Katalysatoren eignen sich Kalium- oder Natriumiodid, bevorzugt Natriumiodid.

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0°C bis 150°C, vorzugsweise von 20°C bis 100°C und Normaldruck.

Die Alkylierung erfolgt im allgemeinen mit Alkylierungsmitteln wie beispielsweise (C₁-C₆)-Alkylhalogeniden, Sulfonsäurestern oder substituierten oder unsubstituierten (C₁-C₆)-Dialkyl- oder (C₁-C₆)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei ]berdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20°C bis +80°C, vorzugsweise von -10°C bis +30°C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium- hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); FE. Frerman et al., J. Biol. Chem. 225, 507 (1982) und N.B. Benoton, K. Kluroda, Int. Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel 11 sind teilweise bekannt oder neu, und können in diesem Fall in Analogie zu bekannten Methoden (vgl. z.B. DE 3 406 329 A1, R.P. Mariella, R. Stansfield, J.Am. Chem. Soc. 73, 1368 (1951) und O. Isler et al., Helv. Chim. Acta 38, 1033 (1955), hergestellt werden.

Die Verbindungen der allgemeinen Formel (111) sind an sich bekannt.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die ertindungsgemäßen substituierten Biphenylpyridone zeigen ein nicht vorhersehbares, wertvolles pharmakologisches Wirkspektrum.

Die erfindungsgemäßen Verbindungen besitzen eine spezifische A ll-antagonistische Wirkung, da sie kompetitiv oder nicht-kompetitiv die Bindung von Angiotensin ll an die Rezeptoren hemmen. Sie unterdrücken die vasokonstriktorischen und Aldosteronsekretions-stimulierenden Effekte des Angiotensin 11. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehimerkrankungen, peripherer Durchblutungsstörungen, Funktionsstörungen der Niere und Neberniere, bronchospastisehen und vaskulär bedingten Erkrankungen der Atemwege, Natriumretention und Ödemen eingesetzt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel

### 6-Butyl-4-methoxycarbonyl-2-oxo-1-{[2'-(N-triphenylmethyl-tetrazol-5-yl)biphenyl-4-yl]methyl}-1,2-dihydropyridin

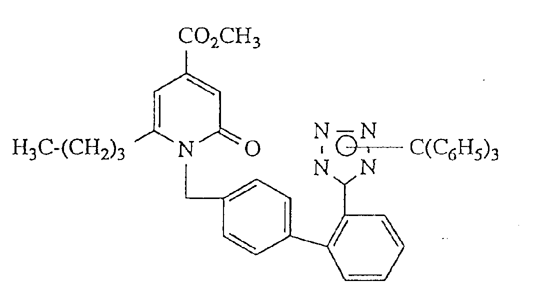

### Verfahren A

20,92 g (0,1 mol) der Verbindung aus Beispiel 1 werden in 200 ml DMF gelöst, portionsweise mit 13,5 g Kaliumtertiärbutylat versetzt und 10 min bei RT gerührt. Dann wird eine Lösung von 55,75 g (0,1 mol) N-Triphenylmethyl-5-[2-(4'-Brommethylbiphenyl)]tetrazol in 200 ml DMF zugetropft und über Nacht bei RT gerührt. Es werden 500 ml Wasser zugetropft, dreimal mit je 300 ml Essigester extrahiert, die vereinigten organischen Phasen über Natriumsulfat getrocknet und eingeengt. Der Rückstand wird mit einem Gradienten aus Petrolether/Essigester (5:1) → (1:2) an 450 g Kieselgel (250-400 mesh) chromatographiert.
Ausbeute: 9,69 g (14 %) farbloser Schaum
R_{f}-Wert: 0,3 Petrolether/Essigester (2:1)

### Verfahren B

Zu einer Lösung von 31,4 g (0,15 mmol) der Verbindung aus Beispiel 1 in 600 ml Dimethoxyethan gibt man 61,1 g (0,188 mol) Caesiumcarbonat, rührt 15 Minuten bei Raumtemperatur, gibt dann 100,4 g (0,18 mol) N-Triphenylmethyl-5-[2-(4'

Brommethyl-biphenyl)]tetrazol zu, rührt über Nacht bei Raumtemperatur und kocht 3 Stunden am Rückfluß.

Dann wird die Reaktionsmischung zwischen Wasser und Essigester (je ca. 0,8 I) verteilt, die organische Phase mit ges. Kochsalzlösung gewaschen, über Na₂S0₄ getrocknet und eingeengt. Der Rückstand wird über 2 kg Kieselgel (230-400 mesh) mit Petrolether/Essigester (5:1) → (1:1) filtriert.
Ausbeute: 39,8 g (38,6 %) gelblicher amorpher Feststoff
R_{f}-Wert: 0,3 Petrolether/Essigester (2:1).

Als Nebenprodukt isoliert man ca. 70 g rohes 6-Butyl-4-methoxycarbonyl-2-{[2'(N-triphenylmethyl-tetrazol-5-yl)biphenyl-4-yl]methoxy-pyridin.
R_{f}-Wert: 0,78 Petrolether/Essigester (2:1).

### Beispiel II

### 6-Butyl-4-methoxycarbonyl-2-oxo-1[(2'-tetrazol-5-yl-biphenyl-4-yl)methyl]-1,2-dihydro-pyridin

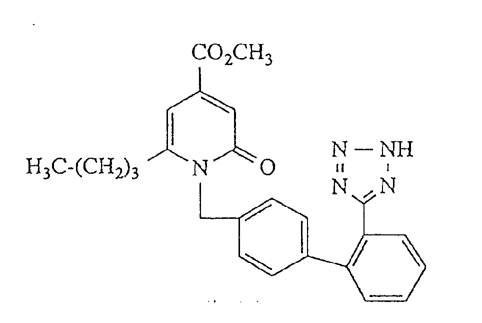

### [Verfahren A]

Eine Lösung von 3,0 g (4,37 mmol) der Verbindung aus Beispiel I in 40 ml Aceton wird mit 0,4 ml 37 %iger Salzsäure 30 min bei RT gerührt und dann ca. 1 min auf dem Wasserbad erwärmt. Nach Zugabe weiterer 0,4 ml 37%iger Salzsäure wird der Prozeß wiederholt.

Man engt zur Trockne ein und chromatographiert den Rückstand an 90 g Kieselgel 230-400 mesh mit Dichlormethan, Dichlormethan/Methanol (50:1) → (10:1). Ausbeute: 1,045 g (54 %) farbloser Schaum

### [Verfahren B]

5 g (7,3 mmol) der Verbindung aus Beispiel I werden in 35 ml Methanol suspendiert und mit 2,5 ml konz. Salzsäure versetzt, wodurch eine Ware Lösung entsteht. Man rührt 3 Stunden bei Raumtemperatur filtriert den ausgefallenen Niederschlag ab, wäscht mit Methanol nach und trocknet im Vakuum über P₂0₅.
Ausbeute: 2,6 g (80,3 %) farbloser Feststoff vom Fp. 209°C (Zers.).
MS (FAB) = 444 (100 %,M + H), 235, (93 %)

### Beispiel III

### 6-Butyl-4-methoxycarbonyl-2-oxo-1)[(2'-tetrazol-5-yl-biphenyl-4-yl)methyl]1,2-dihydro-pyridin Kalium-Salz

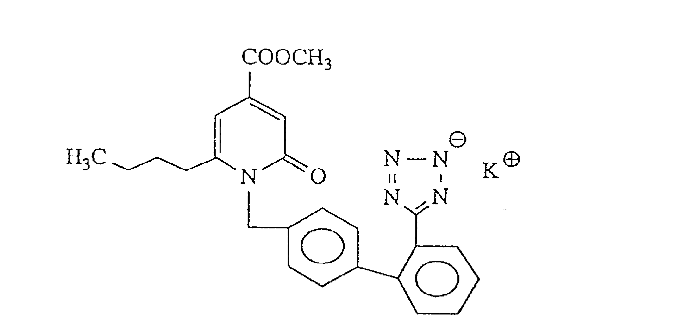

1,33 g (3 mmol) der Verbindung aus Beispiel II werden in 30 ml THF, 30 ml Methanol in der Wärme gelöst, mit 15 ml
Wasser versetzt und bei ca. 5°C 2,85 ml 1 N Kalilauge zugetropft. Man engt zur Trockne ein, rührt den Rückstand in Ether aus, filtriert ab und trocknet über P₂0₅ im Vakuum.
Ausbeute: 1,25 g (86,5 %) farbloser amorpher Feststoff
FAB-MS: 482(100 %, M+H), 520 (20 %, M+K).
¹ H-NMR, [D_{e]}-DMSO δ = 0,8 [t, 3H, (CH₂)₃ CH₃]
   3,35 [s, 3H, COOCH₃]
   5,3 [s, 2H, N-CH₂]

### Beispiele IV

### 6-Butyl-4-carboxy-2-oxo-1[(2'-tetrazol-5-yl-biphenyl-4-yl)methyl]-1,2-dihydropyridin-Dinatriumsalz

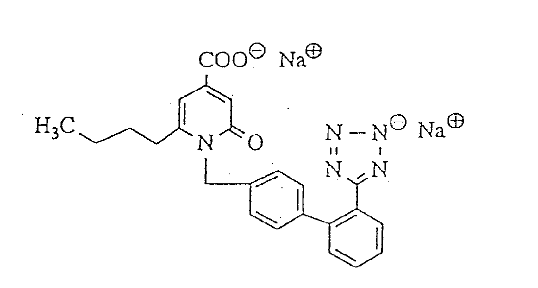

Eine Suspension von 0,66g (1,5 mmol) der Verbindung aus Beispiel II wird in 30 ml THF und 15 ml Wasser mit 3 ml 1 N Natronlauge 2 Stunden bei Raumtemperatur gerührt, wobei eine Klare Lösung resultiert. Es wird zur Trockne eingeengt, der Rückstand in THF/Ether ausgerührt, abgesaugt und über P₂0₅ im Vakuum getrocknet.
Ausbeute: 0,7 g (99 %) Fp. ab 290°C(Zers.)
FAB-MS: 474(70 %, M+H); 452(70 % M-Na+H)

### Beispiel V

### 6-Butyl-4-methoxycarbonyl-2-oxo-1-[(2'-tetrazol-5-yl-biphenyl-4-yl)methyl] 1,2-dihydro-pyridin-Natriumsalz

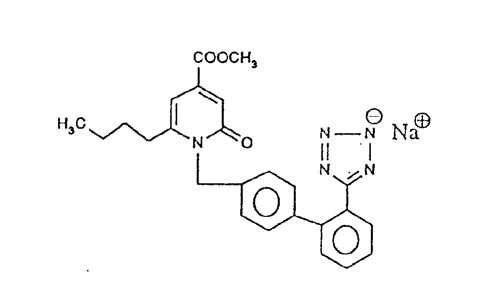

Zu einer Lösung von 443 mg (1 mmol) der Verbindung aus Beispiel II in 10 ml Methanol und 5 ml Aceton gibt man 51 mg (0,95 mmol) Natriummethylat, engt ein und lyophilisiert.

Ausbeute: 460 mg (99 % d.Th.) amorpher Feststoff

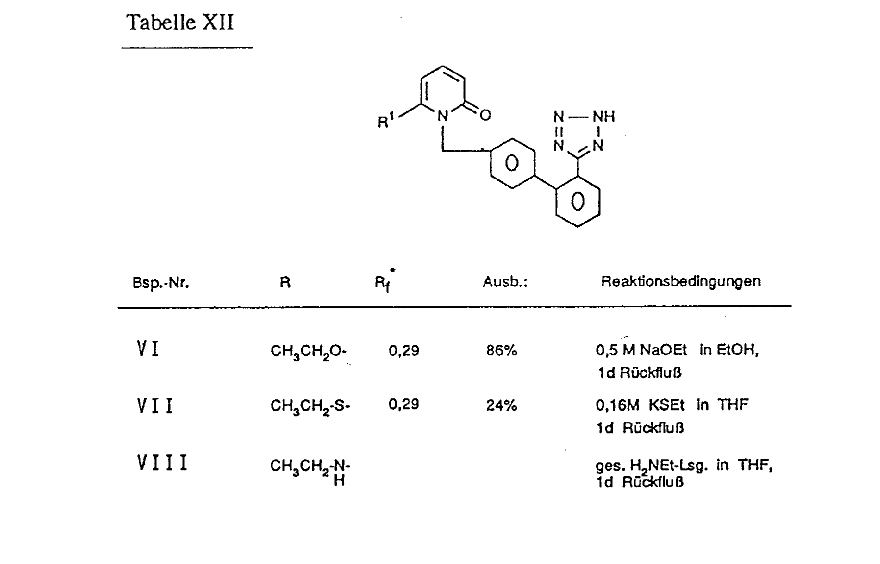

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCI; 2,5 mmol/l CaCl₂ x 2 H₂O; 1,2 mmol/I KH₂P0₄; 10 mmol/l Glucose; 4,8 mmol/l KCI; 1,4 mmol/l MgS0₄ x 7 H₂0 und 25 mmol/l NaHC0₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen/Applikationsvolumen pro Einzelgabe = 100 µl):

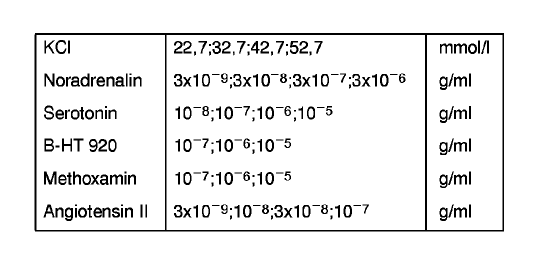

Für die Berechnung der lC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

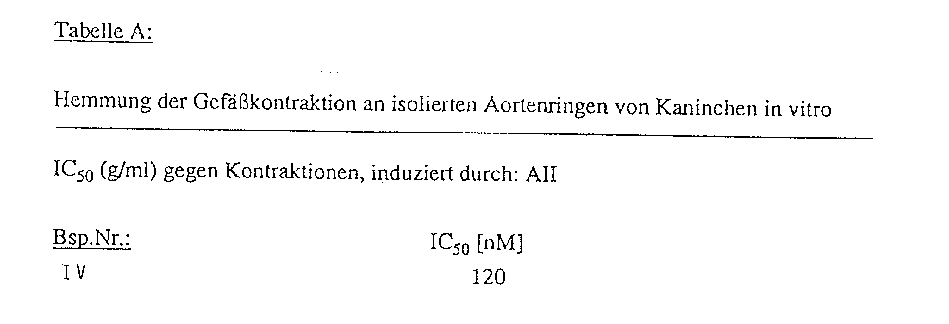

### Blutdruckmessunaen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

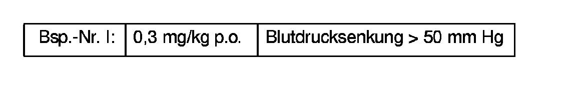

### Bestimmung der antihvpertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform istdie Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin 11-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), 3H-Angiotensin II (3-5 nM), Test-Pufferlösung (50 mM Tris, pH 7,2, 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCI, pH 7,4, 5% PEG 6000) in einem Szintillationscocktail spektrophotometrisch gemessen. Die Analyse der Rohdaten erfolgte mit Computer-Programmen zu Kᵢ- bzw. lC₅₀-Werten (Ki: für die verwendete Radioaktivität korrigierte lC₅₀-Werte; lC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% C0₂ bei 37°C kultiviert. Danach werden die Zellen durch Serumentzug für 2 - 3 Tage synchronisiert und sodann mit All, Serum oder anderen Faktoren zum Wachstum angeregt. Gleichzeitig werden Testverbindungen zugesetzt. Nach 16 - 20 Stunden wird 1 µCi ³H-Thy- midin zugefügt und nach Weiteren 4 Stunden der Einbau dieser Substanz in die TCA-präzipitierbare DNA der Zellen bestimmt.

## Patentansprüche (Patentansprüche für folgende(n) Vertragsstaat(en) : AT, BE, CH, DE, DK, FR, GB, GR, IE, IT, LI, LU, MC, NL, PT, SE)

1. Substituierte Biphenylpyridone der allgemeinen Formel 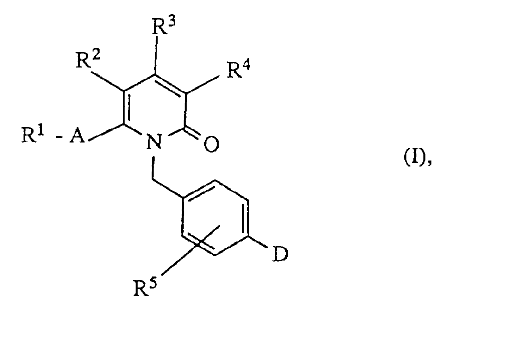 in welcher
R¹ für geradkettiges oder verzweigtes Alkyl mit bis zu 10 Kohlenstoffatomen steht, das gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen substituiert ist, oder für Cycloalkyl mit 3 bis 6 Kohlenstoffatomen oder Halogen steht,
A für ein Sauerstoff- oder Schwefelatom steht oder für die Gruppe der Formel -NR⁶ steht worin
R⁶ Wasserstoff oder geradkettiges oder verzweigtes Alkyl mit bis zu 6 Kohlenstoffatomen bedeutet, oder
R6 gemeinsam mit R¹ unter Einbezug des Stickstoffatoms einen 5-oder 6-gliedrigen, gesättigten oder ungesättigten Heterocyclus bildet,
R2, R3 und R⁴ gleich oder verschieden sind und
für Wasserstoff, Nitro, Cyano, Formyl oder Halogen stehen, oder
für geradkettiges oder verzweigtes Alkyl, Alkenyl, Alknyl, Alkoxy oder Alkylthio mit jeweils bis zu 8 Kohlenstoffatomen stehen, die gegebenenfalls bis zu 2-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Benzyl, Phenyl, Phenoxy, Benzoyl oder durch einen 5- bis 7-gliedrigen, gesättigten oder ungesättigten Heterocyclus mit bis zu 3 Heteroatomen substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 8 Kohlenstoffatomen, Phenoxycarbonyl, Benzyloxycarbonyl oder Carboxy stehen, oder
für Cycloalkyl- oder -alkenyl mit 3 bis 8 Kohlenstoffatomen, für einen 5- bis 7gliedrigem, ungesättigtem Heterocyclus mit bis zu 3 Heteroatomen aus der Reihe S, N oder O,
Phenyl, Phenoxy oder Phenylthio stehen, die gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert sind,
oder,
für Tetrazolyl stehen, die gegebenenfalls durch Methyl oder eine Tritylgruppe substituiert sind oder
für eine Gruppe der Formel 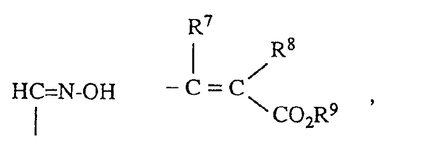
-NR¹⁰R¹¹, -CO-NR¹⁰R¹¹ oder -CH₂-OR¹² stehen
worin
R⁷ Wasserstoff oder geradkettiges oder verzweigtes Alkyl mit bis zu 6 Kohlenstoffatomen bedeutet,
R8 Wasserstoff oder geradkettiges oder verzweigtes Alkyl mit bis zu 8 Kohlenstoffatomen bedeutet, das gegebenenfalls durch Cycloalkyl mit 3 bis 6 Kohlenstoffatomen, Phenyl oder durch einen 5- bis 7-gliedrigen gesättigten oder ungesättigten Heterocyclus mit bis zu 3 Heteroatomen aus der Reihe S, N oder O substituiert ist, wobei die Cyclen gegebenenfalls durch Hydroxy, Hydroxymethyl, Halogen oder durch geradkettiges oder verzweigtes Alkyl oder Alkoxy mit jeweils bis zu 6 Kohlenstoffatomen substituiert sind,
oder Cycloalkyl mit 3 bis 6 Kohlenstoffatomen oder Phenyl bedeutet,
R⁹ Wasserstoff, geradkettiges oder verzweigtes Alkyl mit bis zu 8 Kohlenstoffatomen oder Phenyl bedeutet,
R10 und R¹¹ gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
R¹² geradkettiges oder verzweigtes Acyl mit bis zu 6 Kohlenstoffatomen oder Benzoyl bedeutet,
oder -A-R1 und R² gemeinsam für eine Alkylenkette wird bis zu 5 Kohlenstoffatomen stehen
R⁵ für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen steht,
D für einen Rest der Formel 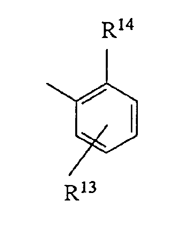 steht,
worin
R¹3 3 die oben angegebene Bedeutung von R⁵ hat und mit dieser gleich oder verschieden ist, und
R¹⁴ eine Gruppe der Formel -CO-R¹⁵, -CO-NR¹⁶R¹⁷ oder -SO₂R¹⁸ bedeutet, worin
R¹⁵ Hydroxy oder geradkettiges oder verzweigtes Alkoxy mit bis zu 6 Kohlenstoffatomen bedeutet,
R¹⁶ und R¹⁷ gleich oder verschieden sind und die oben angegebene Bedeutung von R¹⁰ und R¹¹ haben. oder
R¹⁶ Wasserstoff bedeutet
und
R¹⁷ die Gruppe -SO₂R¹⁸ bedeutet,
R¹⁸ Hydroxy, geradkettiges oder verzweigtes Alkoxy oder Alkyl mit jeweils bis zu 6 Kohlenstoffatomen, Amino oder (C₁-C₆)-Mono- oder -dialkylamino oder Phenyl bedeutet, das gegebenenfalls bis zu 2-fach gleich oder verschieden durch Halogen, Trifluormethyl oder geradkettiges oder verzweigtes Alkyl mit bis zu 4 Kohlenstoffatomen substituiert sein kann, oder
R¹⁴ einen Rest der Formel 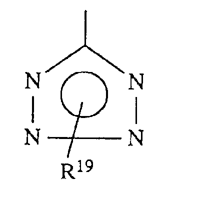 bedeutet,
worin
R¹9 9 Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,
und deren Salze.

2. Substituierte Biphenylpyridone nach Anspruch 1, wobei
R¹ für geradkettiges oder verzweigtes Alkyl mit jeweils bis zu 8 Kohlenstoffatomen steht, das gegebenenfalls durch Cyclopropyl, Cyclopentyl, Cyclohexyl, Hydroxy oder durch gradkettiges oder verzweigtes Alkoxy mit bis zu 4 Kohlenstoffatomen substituiert ist, oder für Cyclopropyl, Cyclopentyl, Cyclohexyl, Fluor, Chlor, Brom oder lod steht,
A für ein Sauerstoff- oder Schwefelatom oder die NH-Gruppe steht,
R2, R3 und R4 gleich oder verschieden sind und
für Wasserstoff, Nitro, Cyano, Formyl, Fluor, Chlor, Brom oder lod stehen, oder für geradkettiges oder verzweigtes Alkyl, Alkenyl, Alkinyl, Alkoxy oder Alkylthio mit jeweils bis zu 6 Kohlenstoffatomen stehen, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, lod oder durch geradkettiges oder verzweigtes Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 6 Kohlenstoffatomen, Phenoxycarbonyl, Benzyloxycarbonyl oder Carboxy stehen, oder
für Cyclopentenyl, Cyclopropyl, Cyclopentyl, Cyclohexyl, Thienyl, Furyl, Phenyl, Phenoxy oder Phenylthio stehen, die gegebenenfalls bis zu 2-fach gleich oder verschieden durch Fluor, Chlor, Brom, lod, Trifluormethyl, Trifluormethoxy, Hydroxymethyl oder durch geradkettiges oder verzweigtes Alkyl oder Alkoxy mit jeweils bis zu 4 Kohlenstoffatomen substituiert sind, oder
für Tetrazolyl stehen, die gegebenenfalls durch Methyl oder eine Tritylgruppe substituiert sind oder
für eine Gruppe der Formel 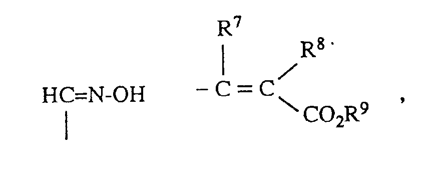 -NR¹⁰R¹¹, -CO-NR¹⁰R¹¹ oder .CH₂-OR¹² stehen,
worin
R⁷ Wasserstoff oder geradkettiges oder verzweigtes Alkyl mit bis zu 4 Kohlenstoffatomen bedeutet,
R⁸ Wasserstoff oder geradkettiges oder verzweigtes Alkyl mit bis zu 6 Kohlenstoffatomen bedeutet, das gegebenenfalls durch Cyclopropyl, Cyclohexyl, Thienyl oder Phenyl substituiert ist, wobei die Cyclen gegebenenfalls durch Hydroxy, Hydroxymethyl oder geradkettiges oder verzweigtes Alkyl oder Alkoxy mit jeweils bis zu 4 Kohlenstoffatomen substituiert sind,
R9 Wasserstoff, geradkettiges oder verzweigtes Alkyl mit bis zu 6 Kohlenstoffatomen bedeutet,
R¹⁰ und R¹¹ gleich, oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
R12 ² geradkettiges oder verzweigtes Acyl mit bis zu 6 Kohlenstoffatomen oder Benzoyl bedeutet,
oder -A-R¹ und R² gemeinsam für eine Alkylenkette mit bis zu 5 Kohlenstoffatomen stehen,
R5 für Wasserstoff, Fluor, Chlor, Brom, Hydroxy, Trifluormethyl, Trifluormethoxy oder für geradkettiges oder verzweigtes Alkyl oder Alkoxy mit jeweils bis zu 4 Kohlenstoffatomen steht,
D für einen Rest der Formel 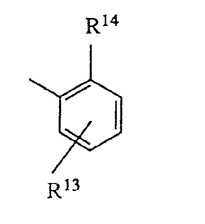 steht,
worin
R¹ 3 die oben angegebene Bedeutung von R⁵ hat und mit dieser gleich oder verschieden ist und
R¹⁴ eine Gruppe der Formel -CO-R¹⁵, -CO-NR¹⁶R¹⁷ oder -S0₂R¹⁸ bedeutet,
worin
R¹⁵ Hydroxy oder geradkettiges oder verzweigtes Alkoxy mit bis zu 4 Kohlenstoffatomen bedeutet,
R¹⁶ und R¹⁷ gleich oder verschieden sind und die oben angegebene Bedeutung von R¹⁰ und R¹¹ haben oder
R¹⁶ Wasserstoff bedeutet und
R¹⁷ die Gruppe -SO₂R¹⁸ bedeutet,
R¹⁸ geradkettiges oder verzweigtes Alkyl mit bis zu 4 Kohlenstoffatomen oder p-Tolyl bedeutet, oder
_{R1}4 einen Rest der Formel 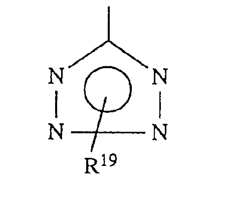 bedeutet,
worin
R¹9 9 Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

3. Substituierte Biphenylpyridone der Formel 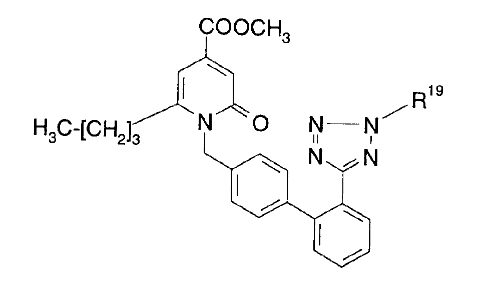 worin
_{R}1₉ für Wasserstoff oder
für den Triphenylmethylrest steht
und deren Salze.

4. 6-Butyl-4-methoxycarbonyl-2-oxo-1-[(2'-tetrazol-yl-biphenyl-4-yl)methyl]-1,2-dihydropyridin nach Anspruch 3 und dessen Salze.

5. Substituierte Biphenylpyridone nach Anspruch 1 bis 4 zur therapeutischen Behandlung.

6. Verfahren zur Herstellung von substituierten Biphenylpyridonen nach Anspruch 1 bis 4 dadurch gekennzeichnet, daß man Pyridone der allgemeinen Formel (II) 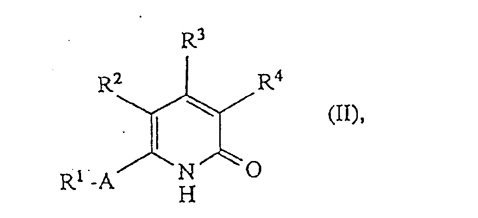 in welcher
R¹, R², R⁴ und A die angegebene Bedeutung haben,
mit Verbindungen der allgemeinen Formel (III) 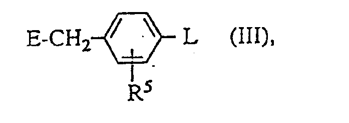 in welcher
E für Halogen, vorzugsweise für Brom steht und
L für einen Rest der Formel 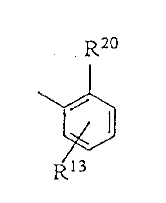 steht,
worin
R¹3 3 die oben angegebene Bedeutung hat und
R20 C₁-C₄-Alkoxycarbonyl oder
einen Rest der Formel 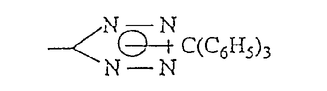 bedeutet,
in inerten Lösemitteln, in Anwesenheit einer Base und gegebenenfalls unter Zusatz eines Katalysators umsetzt,
und anschließend im Fall des Tetrazolrestes die Triphenylmethylgruppe mit Säuren in organischen Lösemitteln und/oder Wasser nach üblichen Bedingungen abspaltet,
und gegebenenfalls im Fall der unter den Substituenten R¹⁴ und/oder R²⁰ aufgeführten carbonylischen Reste nach Verseifung der jeweiligen Ester beispielsweise durch Amidierung oder Sulfoamidierung nach üblichen Methoden derivatisiert,
und gegebenenfalls auch die Substituenten R², R³, R⁴, R⁵, R¹³ und R¹⁹ nach bekannten Methoden variiert.

7. Verfahren nach Anspruch 6 dadurch gekennzeichnet, daß man die Umsetzung in einem Temperaturbereich von - 100°C bis + 100°C durchführt.

8. Arzneimittel enthaltend mindestens ein substituiertes Biphenylpyridon nach Anspruch 1 bis 4.

9. Arzneimittel nach Anspruch 8 zur Behandlung von Bluthochdruck und Atherosklerose.

10. Verfahren zur Herstellung eines Arzneimittels nach Anspruch 8 und 9 dadurch gekennzeichnet, daß man die substituierten Biphenylpyridone gegebenenfalls mit der Hilfe von geeigneten Hilfs- und Trägerstoffen in eine geeignete Applikationsform bringt.

11. Verwendung von substituierten Biphenylpyridonen nach Anspruch 1 bis 4 zur Herstellung von Arzneimitteln.

12. Verwendung von substituierten Biphenylpyridonen nach Anspruch 1 bis 4 zur Herstellung von Arzneimitteln zur Behandlung von Bluthochdruck und Atherosklerose.

## Patentansprüche (Patentansprüche für folgende(n) Vertragsstaat(en) : ES)

1. Verfahren zur Herstellung von substituierten Biphenylpyridonen der Formel (I) 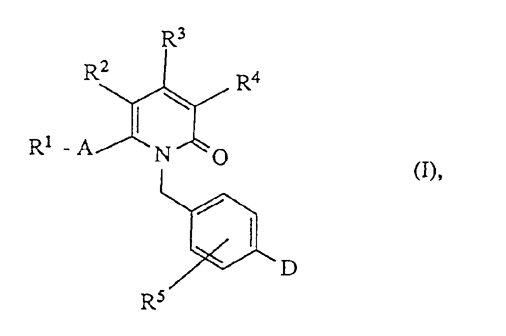 in welcher
R¹ für geradkettiges oder verzweigtes Alkyl mit bis zu 10 Kohlenstoffatomen steht, das gegebenenfalls durch Cycloalkyl mit 3 bis 6 Kohlenstoffatomen, Hydroxy oder durch geradkettiges oder verzweigtes Alkoxy mit bis zu 6 Kohlenstoffatomen substituiert ist, oder für Cycloalkyl mit 3 bis 6 Kohlenstoffatomen oder Halogen steht,
A für ein Sauerstoff- oder Schwefelatom steht oder für die Gruppe der Formel -NR⁶ steht worin
R⁶ Wasserstoff oder geradkettiges oder verzweigtes Alkyl mit bis zu 6 Kohlenstoffatomen bedeutet, oder
R6 gemeinsam mit R¹ unter Einbezug des Stickstoffatoms einen 5-oder 6-gliedrigen, gesättigten oder ungesättigten Heterocyclus bildet,
R2, R3 und R4 gleich oder verschieden sind und
für Wasserstoff, Nitro, Cyano, Formyl oder Halogen stehen, oder
für geradkettiges oder verzweigtes Alkyl, Alkenyl, Alkinyl, Alkoxy oder Alkylthio mit jeweils bis zu 8 Kohlenstoffatomen stehen, die gegebenenfalls bis zu 2-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Benzyl, Phenyl, Phenoxy, Benzoyl oder durch einen 5- bis 7-gliedrigen, gesättigten oder ungesättigten Heterocyclus mit bis zu 3 Heteroatomen substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweig[es Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 8 Kohlenstoffatomen, Phenoxycarbonyl, Benzyloxycarbonyl oder Carboxy stehen, oder
für Cycloalkyl- oder -alkenyl mit 3 bis 8 Kohlenstoffatomen, für einen 5- bis 7gliedrigem, ungesättigtem Heterocyclus mit bis zu 3 Heteroatomen aus der Reihe S, N oder O,
Phenyl, Phenoxy oder Phenylthio stehen, die gegebenenfalls bis zu 3-fach gleich oder verschieden durch Halogen, Nitro, Cyano, Hydroxy, Hydroxymethyl, Trifluormethyl, Trifluormethoxy oder durch geradkettiges oder verzweigtes Alkyl oder Alkoxy mit jeweils bis zu 6 Kohlenstoffatomen substituiert sind,
oder,
für Tetrazolyl stehen, die gegebenenfalls durch Methyl oder eine Tritylgruppe substituiert sind oder
für eine Gruppe der Formel 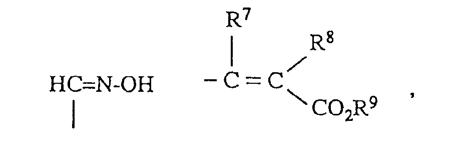
-NR¹⁰R¹¹, -CO-NR¹⁰R¹¹ oder -CH₂-OR¹² stehen
worin
R⁷ Wasserstoff oder geradkettiges oder verzweigtes Alkyl mit bis zu 6 Kohlenstoffatomen bedeutet,
R⁸ Wasserstoff oder geradkettiges oder verzweigtes Alkyl mit bis zu 8 Kohlenstoffatomen bedeutet, das gegebenenfalls durch Cycloalkyl mit 3 bis 6 Kohlenstoffatomen, Phenyl oder durch einen 5- bis 7-gliedrigen gesättigten oder ungesättigten Heterocyclus mit bis zu 3 Heteroatomen aus der Reihe S, N oder 0 substituiert ist, wobei die Cyclen gegebenenfalls durch Hydroxy, Hydroxymethyl, Halogen oder durch geradkettiges oder verzweigtes Alkyl oder Alkoxy mit jeweils bis zu 6 Kohlenstoffatomen substituiert sind, oder Cycloalkyl mit 3 bis 6 Kohlenstoffatomen oder Phenyl bedeutet,
R9 Wasserstoff, geradkettiges oder verzweigtes Alkyl mit bis zu 8 Kohlenstoffatomen oder Phenyl bedeutet,
R¹ und R¹¹ gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
R¹ 2 geradkettiges oder verzweigtes Acyl mit bis zu 6 Kohlenstoffatomen oder Benzoyl bedeutet, oder -A-R¹ und R² gemeinsam für eine Alkylenkette wild bis zu 5 Kohlenstoffatomen stehen
R⁵ für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen steht,
D für einen Rest der Formel 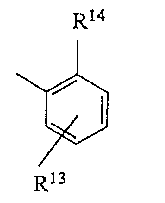 steht,
worin
R¹ 3 die oben angegebene Bedeutung von R⁵ hat und mit dieser gleich oder verschieden ist, und
R14 eine Gruppe der Formel -CO-R15, -CO-NR¹⁶R¹⁷ oder -SO₂R¹⁸ bedeutet, worin
R¹⁵ Hydroxy oder geradkettiges oder verzweigtes Alkoxy mit bis zu 6 Kohlenstoffatomen bedeutet,
R¹⁶ und R¹⁷ gleich oder verschieden sind und die oben angegebene Bedeutung von R¹⁰ und R¹¹ haben, oder
R¹⁶ Wasserstoff bedeutet
und
R¹⁷ die Gruppe -SO₂R¹⁸ bedeutet,
R¹⁸ Hydroxy, geradkettiges oder verzweigtes Alkoxy oder Alkyl mit jeweils bis zu 6 Kohlenstoffatomen, Amino oder (C₁-C₆)-Mono- oder -dialkylamino oder Phenyl bedeutet, das gegebenenfalls bis zu 2-fach gleich oder verschieden durch Halogen, Trifluormethyl oder geradkettiges oder verzweigtes Alkyl mit bis zu 4 Kohlenstoffatomen substituiert sein kann, oder
R¹⁴ einen Rest der Formel 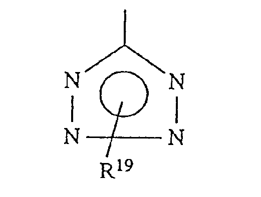 bedeutet,
worin
R¹⁹ Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,
und deren Salze,
dadurch gekennzeichnet, daß man Pyridone der allgemeinen Formel (II) 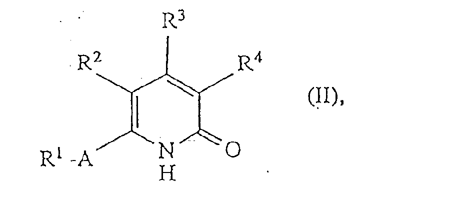 in welcher
R¹, R², R⁴ und A die angegebene Bedeutung haben, mit Verbindungen der allgemeinen Formel (III) 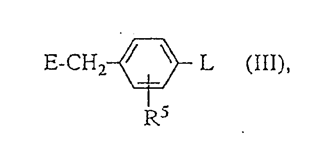 in welcher
E für Halogen, vorzugsweise für Brom steht und
L für einen Rest der Formel 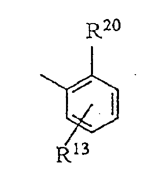 steht,
worin
R¹³ die oben angegebene Bedeutung hat und
R20 C₁-C₄-Alkoxycarbonyl oder
einen Rest der Formel 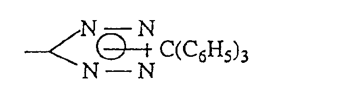 bedeutet,
in inerten Lösemittein, in Anwesenheit einer Base und gegebenenfalls unter Zusatz eines Katalysators umsetzt,
und anschließend im Fall des Tetrazolrestes die Triphenylmethylgruppe mit Säuren in organischen Lösemitteln und/oder Wasser nach üblichen Bedingungen abspaltet,
und gegebenenfalls im Fall der unter den Substituenten R¹⁴ und/oder R²0 aufgeführten carbonylischen Reste nach Verseifung der jeweiligen Ester beispielsweise durch Amidierung oder Sulfoamidierung nach üblichen Methoden derivatisiert,
und gegebenenfalls auch die Substituenten R², R3, R⁴, R⁵, R¹3 und R19 nach bekannten Methoden variiert

2. Verfahren nach Anspruch 1 zur Herstellung von substituierten Biphenylpyridonen der Formel (I) in welcher
R1 für geradkettiges oder verzweigtes Alkyl mitjeweils bis zu 8 Kohlenstoffatomen steht, das gegebenenfalls durch Cyclopropyl, Cyclopentyl, Cyclohexyl, Hydroxy oder durch gradkettiges oder verzweigtes Alkoxy mit bis zu 4 Kohlenstoffatomen substituiert ist, oder für Cyclopropyl, Cyclopentyl, Cyclohexyl, Fluor, Chlor, Brom oder lod steht,
A für ein Sauerstoff- oder Schwefelatom oder die NH-Gruppe steht,
R2, R3 und R4 gleich oder verschieden sind und
für Wasserstoff, Nitro, Cyano, Formyl, Fluor, Chlor, Brom oder lod stehen, oder
für geradkettiges oder verzweigtes Alkyl, Alkenyl, Alkinyl, Alkoxy oder Alkylthio mit jeweils bis zu 6 Kohlenstoffatomen stehen, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, lod oder durch geradkettiges oder verzweigtes Alkyl oder Alkoxy mit jeweils bis zu 6 Kohlenstoffatomen substituiert sein können, oder
für geradkettiges oder verzweigtes Acyl oder Alkoxycarbonyl mit jeweils bis zu 6 Kohlenstoffatomen, Phenoxycarbonyl, Benzyloxycarbonyl oder Carboxy stehen, oder
für Cyclopentenyl, Cyclopropyl, Cyclopentyl, Cyclohexyl, Thienyl, Furyl, Phenyl, Phenoxy oder Phenylthio stehen, die gegebenenfalls bis zu 2-fach gleich oder verschieden durch Fluor, Chlor, Brom, lod, Trifluormethyl, Trifluormethoxy, Hydroxymethyl oder durch geradkettiges oder verzweigtes Alkyl oder Alkoxy mit jeweils bis zu 4 Kohlenstoffatomen substituiert sind, oder
für Tetrazolyl stehen, die gegebenenfalls durch Methyl oder eine Tritylgruppe substituiert sind oder 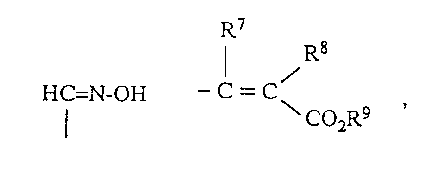
für eine Gruppe der Formel
-NR¹⁰R¹¹, -CO-NR¹⁰R¹¹ oder -CH₂-OR¹² stehen,
worin
R⁷ Wasserstoff oder geradkettiges oder verzweigtes Alkyl mit bis zu 4 Kohlenstoffatomen bedeutet,
R8 Wasserstoff oder geradkettiges oder verzweigtes Alkyl mit bis zu 6 Kohlenstoffatomen bedeutet, das gegebenenfalls durch Cyclopropyl, Cyclohexyl, Thienyl oder Phenyl substituiert ist, wobei die Cyclen gegebenenfalls durch Hydroxy, Hydroxymethyl oder geradkettiges oder verzweigtes Alkyl oder Alkoxy mit jeweils bis zu 4 Kohlenstoffatomen substituiert sind,
R9 Wasserstoff, geradkettiges oder verzweigtes Alkyl mit bis zu 6 Kohlenstoffatomen bedeutet,
R¹⁰ und R¹¹ gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
R¹² geradkettiges oder verzweigtes Acyl mit bis zu 6 Kohlenstoffatomen oder Benzoyl bedeutet,
oder -A-R¹ und R² gemeinsam für eine Alkylenkette mit bis zu 5 Kohlenstoffatomen stehen,
R5 für Wasserstoff, Fluor, Chlor, Brom, Hydroxy, Trifluormethyl, Trifluormethoxy oder für geradkettiges oder verzweigtes Alkyl oder Alkoxy mit jeweils bis zu 4 Kohlenstoffatomen steht,
D für einen Rest der Formel 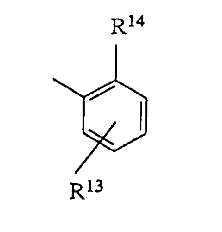 steht,
worin
R¹³ die oben angegebene Bedeutung von R⁵ hat undmit dieser gleich oder verschieden ist und
R¹⁴ eine Gruppe der Formel -CO-R¹⁵, -CO-NR¹6R¹⁷ oder -SO₂R¹⁸ bedeutet, worin
R¹⁵ Hydroxy oder geradkettiges oder verzweigtes Alkoxy mit bis zu 4 Kohlenstoffatomen bedeutet,
R¹⁶ und R¹⁷ gleich oder verschieden sind und die oben angegebene Bedeutung von R¹⁰ und R¹¹ haben oder
R¹⁶ Wasserstoff bedeutet und
R¹⁷ die Gruppe -SO₂R¹⁸ bedeutet,
R¹8 geradkettiges oder verzweigtes Alkyl mit bis zu 4 Kohlenstoffatomen oder p-Tolyl bedeutet, oder
R¹⁴ einen Rest der Formel 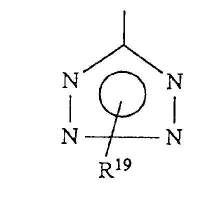 bedeutet,
worin
R¹9 Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

3. Verfahren zur Herstellung von substituierten Biphenylpyridonen der Formel 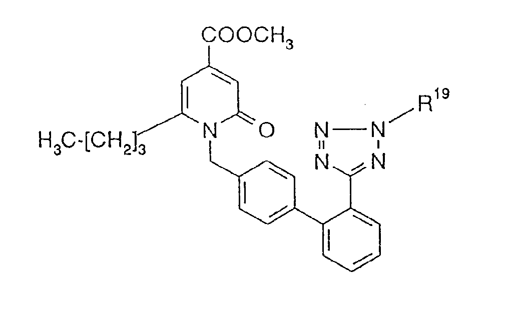 worin
R¹⁹ ₉ für Wasserstoff oder
für den Triphenylmethylrest steht
und deren Salze,
dadurch gekennzeichnet, daß man Pyridone der Formel (II) 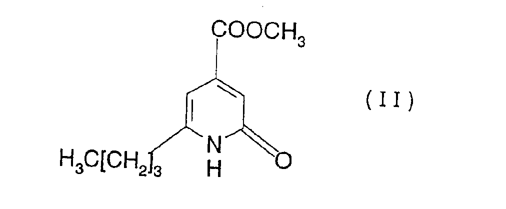 mit Verbindungen der allgemeinen Formel (III) 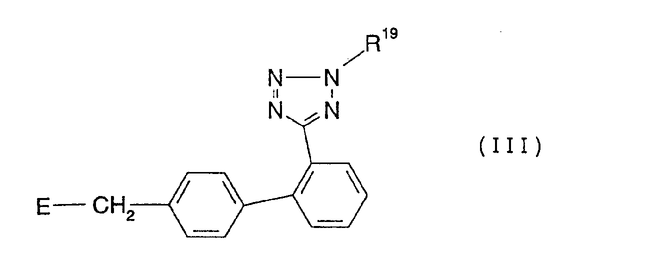 in welcher
E für Halogen, Vorzugsweise für Brom steht und in inerten Lösemitteln, in Anwesenheit einer Base und gegebenenfalls unter Zusatz eines Katalysators umsetzt,
und anschließend im Fall des Tetrazolrestes die Triphenylmethylgruppe mit Säuren in organischen Lösemitteln und/oder Wasser nach üblichen Bedingungen abspaltet.

4. Verfahren nach Anspruch 3 zur Herstellung von 6-Buthyl-4-methoxycarbonyl-2-oxo-1-[2'-tetrazol-yl-biphenyl-4-yl)methyl]-1,2-dihydropyridin und dessen Salze.

5. Verfahren nach Anspruch 1 bis 4 dadurch gekennzeichnet, daß man die Umsetzung in einem Temperaturbereich von - 100°C bis + 100°C durchführt.

## Claims (Claims for the following Contracting State(s) : AT, BE, CH, DE, DK, FR, GB, GR, IE, IT, LI, LU, MC, NL, PT, SE)

1. Substituted biphenylpyridones of the general formula 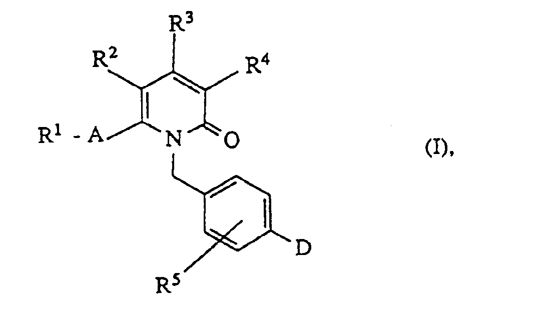 in which
R¹ represents straight-chain or branched alkyl having up to 10 carbon atoms, which is optionally substituted by cycloalkyl having 3 to 6 carbon atoms, hydroxyl or by straight-chain or branched alkoxy having up to 6 carbon atoms, or represents cycloalkyl having 3 to 6 carbon atoms or halogen,
A represents an oxygen or sulphur atom or represents the group of the formula -NR6 in which
R6 denotes hydrogen or straight-chain or branched alkyl having up to 6 carbon atoms, or
R6, together with R¹ including the nitrogen atom, forms a 5- or 6-membered, saturated or unsaturated heterocycle,
R², R3 and R⁴ are identical or different and represent hydrogen, nitro, cyano, formyl or halogen, or
represent straight-chain or branched alkyl, alkenyl, alkinyl, alkoxy or alkylthio each having up to 8 carbon atoms, each of which is optionally substituted up to 2 times by identical or different substituents from the group comprising hydroxyl, cyano, halogen, carboxyl, straight-chain or branched alkoxy, acyl or alkoxycarbonyl each having up to 6 carbon atoms, or by benzyl, phenyl, phenoxy or benzoyl or by a 5- to 7-membered, saturated or unsaturated heterocycle having up to 3 heteroatoms, where the cycles can in turn be substituted up to 2 times by identical or different substituents from the group comprising trifluoromethyl, trifluoromethoxy, halogen, nitro, cyano, hydroxyl, hydroxymethyl or by straight-chain or branched alkyl or alkoxy each having up to 6 carbon atoms, or
represent straight-chain or branched acyl or alkoxycarbonyl each having up to 8 carbon atoms, phenoxycarbonyl, benzyloxycarbonyl or carboxyl, or
represent cycloalkyl or -alkenyl having 3 to 8 carbon atoms, or a 5- to 7-membered, unsaturated heterocycle having up to 3 heteroatoms from the series comprising S, N or O,
phenyl, phenoxy and phenylthio, each of which is optionally substituted up to 3 times by identical or different substituents from the group comprising halogen, nitro, cyano, hydroxyl, hydroxymethyl, trifluoromethyl and trifluoromethoxy or by straight-chain or branched alkyl or alkoxy each having up to 6 carbon atoms, or,
represent tetrazolyl, each of which is optionally substituted by methyl or a trityl group, or represent a group of the formula 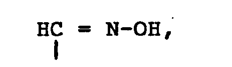 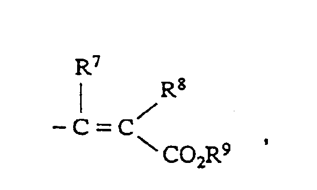 -NR¹⁰R¹¹ -CO-NR¹⁰R¹¹ or-CH₂-OR¹² in which
R⁷ denotes hydrogen or straight-chain or branched alkyl having up to 6 carbon atoms,
R8 denotes hydrogen or straight-chain or branched alkyl having up to 8 carbon atoms, which is optionally substituted by cycloalkyl having 3 to 6 carbon atoms, phenyl or by a 5- to 7-membered saturated or unsaturated heterocycle having up to 3 heteroatoms from the series comprising S, N and 0, where the cycles are optionally substituted by hydroxyl, hydroxymethyl or halogen or by straight-chain or branched alkyl or alkoxy each having up to 6 carbon atoms, or denotes cycloalkyl having 3 to 6 carbon atoms or phenyl,
R⁹ denotes hydrogen, straight-chain or branched alkyl having up to 8 carbon atoms or phenyl,
R¹⁰ and R¹¹ are identical or different and denote hydrogen, cycloalkyl having 3 to 8 carbon atoms or straight-chain or branched alkyl having up to 8 carbon atoms, which is optionally substituted by phenyl,
R¹² denotes straight-chain or branched acyl having up to 6 carbon atoms or benzoyl, or -A-R¹ and R² together represent an alkylene chain having up to 5 carbon atoms,
R⁵ represents hydrogen, halogen, cyano, nitro, trifluoromethyl, hydroxyl, trifluoromethoxy or straight-chain or branched alkyl or alkoxy each having up to 6 carbon atoms,
D represents a radical of the formula 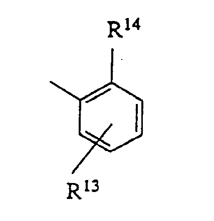 in which
R¹3 3 has the abovementioned meaning of R⁵ and is identical to or different from this, and
R¹⁴ denotes a group of the formula -CO-R¹⁵, -CO-NR¹⁶R¹⁷ or -S_{O2}R¹⁸, in which
R¹⁵ denotes hydroxyl or straight-chain or branched alkoxy having up to 6 carbon atoms,
R¹6 and R¹⁷ are identical or different and have the abovementioned meaning of R¹⁰ and R¹¹, or
R16 denotes hydrogen and
R¹⁷ denotes the group -S0₂R^{1 8},
R¹8 denotes hydroxyl, straight-chain or branched alkoxy or alkyl each having up to 6 carbon atoms, amino or (C₁-C₆)-mono- or -dialkylamino or phenyl which can optionally be substituted up to 2 times by identical or different substituents from the group comprising halogen, trifluoromethyl and straight-chain or branched alkyl having up to 4 carbon atoms, or
R14 4 denotes a radical of the formula 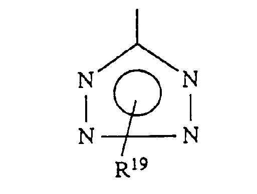 in which
R¹⁹ denotes hydrogen or straight-chain or branched alkyl having up to 8 carbon atoms, which is optionally substituted by straight-chain or branched acyl having up to 6 carbon atoms or denotes the triphenylmethyl group,
and their salts.

2. Substituted biphenylpyridones according to Claim 1, in which
R¹ represents straight-chain or branched alkyl, each having up to 8 carbon atoms, which is optionally substituted by cyclopropyl, cyclopentyl, cyclohexyl or hydroxyl or by straight-chain or branched alkoxy having up to 4 carbon atoms, or represents cyclopropyl, cyclopentyl, cyclohexyl, fluorine, chlorine, bromine or iodine,
A represents an oxygen or sulphur atom, or the NH group
R2, R3 and R4 are identical or different and represent hydrogen, nitro, cyano, formyl, fluorine, chlorine, bromine or iodine, or represent straight-chain or branched alkyl, alkenyl, alkinyl, alkoxy, or alkylthio each having up to 6 carbon atoms, each of which is optionally substituted by hydroxyl, cyano, fluorine, chlorine, bromine, carboxyl, straight-chain or branched alkoxy, acyl or alkoxycarbonyl each having up to 4 carbon atoms, or by benzyl, phenyl, phenoxy, benzoyl or thienyl, where the cycles can in turn be substituted by trifluoromethoxy, trifluoromethyl, hydroxymethyl, fluorine, chlorine, bromine, iodine or by straight-chain or branched alkyl or alkoxy each having up to 6 carbon atoms, or
represent straight-chain or branched acyl or alkoxycarbonyl each having up to 6 carbon atoms, phenoxycarbonyl, benzyloxycarbonyl or carboxyl, or
represent cyclopentenyl, cyclopropyl, cyclopentyl, cyclohexyl, thienyl, furyl, phenyl, phenoxy or phenylthio, each of which is optionally substituted up to 2 times by identical or different substituents from the group comprising fluorine, chlorine, bromine, iodine, trifluoromethyl, trifluoromethoxy and hydroxymethyl or by straight-chain or branched alkyl or alkoxy each having up to 4 carbon atoms,
or
represent tetrazolyl, each of which is optionally substituted by methyl or a trityl group, or represent a group of the formula 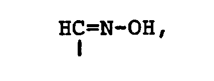 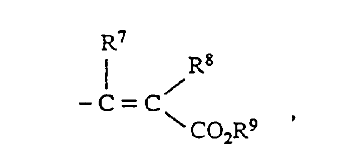 -NR¹⁰R¹¹, -CO-NR¹⁰⁻R¹¹ or -CH₂-OR¹², in which
R⁷ denotes hydrogen or straight-chain or branched alkyl having up to 4 carbon atoms,
R8 denotes hydrogen or straight-chain or branched alkyl having up to 6 carbon atoms, which is optionally substituted by cyclopropyl, cyclohexyl, thienyl or phenyl, where the cycles are optionally substituted by hydroxyl, hydroxymethyl or straight-chain or branched alkyl or alkoxy each having up to 4 carbon atoms,
R9 denotes hydrogen, straight-chain or branched alkyl having up to 6 carbon atoms,
R¹⁰ and R¹¹ are identical or different and denote hydrogen, cyclopropyl, cyclopentyl, cyclohexyl or straight-chain or branched alkyl having up to 6 carbon atoms, which is optionally substituted by phenyl,
R12 denotes straight-chain or branched acyl having up to 6 carbon atoms or benzoyl, or -A-R¹ and R² together represent an alkylene chain having up to 5 carbon atoms,
R⁵ represents hydrogen, fluorine, chlorine, bromine, hydroxyl, trifluoromethyl, trifluoromethoxy or straight-chain or branched alkyl or alkoxy each having up to 4 carbon atoms,
D represents a radical of the formula 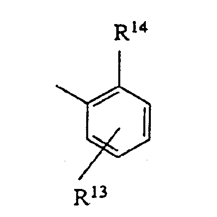 in which
R¹³ has the abovementioned meaning of R⁵ and is identical to or different from this and
R¹⁴ denotes a group of the formula -CO-R¹⁵, -CO-NR16R17 or -S0₂R¹⁸, in which
R¹⁵ denotes hydroxyl or straight-chain or branched alkoxy having up to 4 carbon atoms,
R¹⁶ and R¹⁷ are identical or different and have the abovementioned meaning of R¹⁰ and R¹¹ or
_{R}16 denotes hydrogen and
R¹⁷ denotes the group -SO₂R¹⁸,
R¹⁸ denotes straight-chain or branched alkyl having up to 4 carbon atoms or p-tolyl, or
R¹⁴ denotes a radical of the formula in which 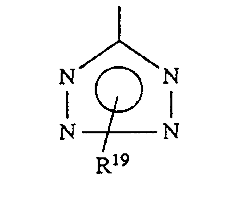
R¹⁹ denotes hydrogen or straight-chain or branched alkyl having up to 6 carbon atoms, which is optionally substituted by straight-chain or branched acyl having up to 4 carbon atoms or denotes the triphenylmethyl group,
and their salts.

3. Substituted biphenylpyridones of the formula 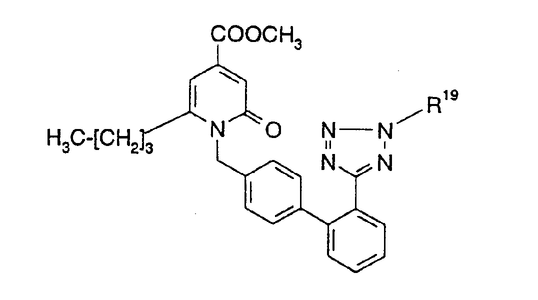 in which
R¹⁹ represents hydrogen or represents the triphenylmethyl radical,
and their salts.

4. 6-Butyl-4-methoxycarbonyl-2-oxo-1-[(2'-tetrazol-yl-biphenyl-4-yl)methyl]-1,2-dihydropyridine according to Claim 3, and its salts.

5. Substituted biphenylpyridones according to Claims 1 to 4 for therapeutic treatment.

6. Process for the preparation of substituted biphenylpyridones according to Claims 1 to 4, characterised in that pyridones of the general formula (II) 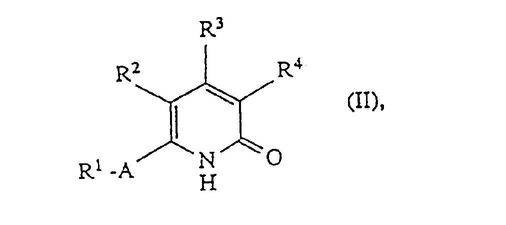 in which
R¹, R2, R4 and A have the given meaning,
are reacted with compounds of the general formula (III) 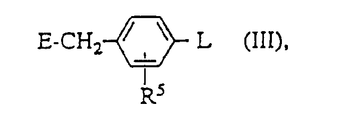 in which
E represents halogen, preferably bromine, and
L represents a radical of the formula 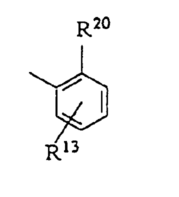 in which
R¹³ has the abovementioned meaning and
R²0 denotes C₁-C₄-alkoxycarbonyl or a radical of the formula 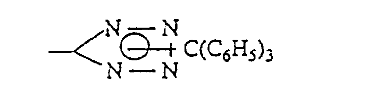 in inert solvents, in the presence of a base and if appropriate with the addition of a catalyst,
and then in the case of the tetrazole radical the triphenylmethyl group is removed with acids in organic solvents and/or water according to customary conditions,
and if appropriate in the case of the carbonyl radicals mentioned under the substituents R¹⁴ and/or R²0, derivatised after hydrolysis of the respective esters, for example, by amidation or sulphonamidation according to customary methods,
and, if appropriate, the substituents R², R3, R⁴, R⁵, R¹3 and R¹⁹ are also varied according to the known methods.

7. Process according to Claim 6, characterised in that the reaction is carried out in a temperature range from -100°C to+100°C.

8. Medicaments containing at least one substituted biphenylpyridone according to Claims 1 to 4.

9. Medicaments according to Claim 8 for the treatment of high blood pressure and atherosclerosis.

10. Process for the preparation of a medicament according to Claims 8 and 9, characterised in that the substituted biphenylpyridones are brought into a suitable administration form, if appropriate with the aid of suitable auxiliaries and excipients.

11. Use of substituted biphenylpyridones according to Claims 1 to 4 for the production of medicaments.

12. Use of substituted biphenylpyridones according to Claims 1 to 4 for the production of medicaments for the treatment of high blood pressure and atherosclerosis.

## Claims (Claims for the following Contracting State(s) : ES)

1. Process for the preparation of substituted biphenylpyridones of the formula (I) 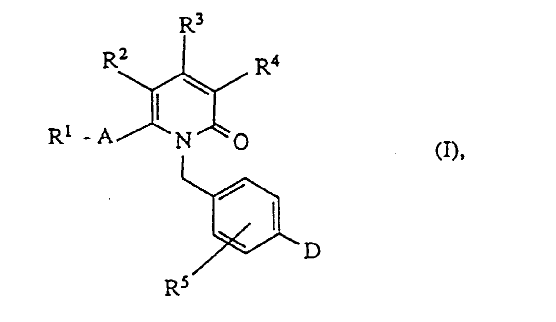 in which
R¹ represents straight-chain or branched alkyl having up to 10 carbon atoms, which is optionally substituted by cycloalkyl having 3 to 6 carbon atoms,hydroxyl or by straight-chain or branched alkoxy having up to 6 carbon atoms,or represents cycloalkyl having 3 to 6 carbon atoms or halogen,
A represents an oxygen or sulphur atom or represents the group of the formula -NR⁶ in which
R6 denotes hydrogen or straight-chain or branched alkyl having up to 6 carbon atoms, or
R6, together with R¹ including the nitrogen atom, forms a 5- or 6-membered, saturated or unsaturated heterocycle,
R², R3 and R⁴ are identical or different and represent hydrogen, nitro, cyano, formyl or halogen, or represent straight-chain or branched alkyl, alkenyl, alkinyl, alkoxy or alkylthio each having up to 8 carbon atoms, each of which is optionally substituted up to 2 times by identical or different substituents from the group comprising hydroxyl, cyano, halogen, carboxyl, straight-chain or branched alkoxy, acyl or alkoxycarbonyl each having up to 6 carbon atoms, or by benzyl, phenyl, phenoxy or benzoyl or by a 5- to 7-membered, saturated or unsaturated heterocycle having up to 3 heteroatoms, where the cycles can in turn be substituted up to 2 times by identical or different substituents from the group comprising trifluoromethyl, trifluoromethoxy, halogen, nitro, cyano, hydroxyl, hydroxymethyl or by straight-chain or branched alkyl or alkoxy each having up to 6 carbon atoms, or
represent straight-chain or branched acyl or alkoxycarbonyl each having up to 8 carbon atoms, phenoxycarbonyl, benzyloxycarbonyl or carboxyl, or
represent cycloalkyl or -alkenyl having 3 to 8 carbon atoms, or a 5- to 7-membered, unsaturated heterocycle having up to 3 heteroatoms from the series comprising S, N or 0, phenyl, phenoxy and phenylthio, each of which is optionally substituted up to 3 times by identical or different substituents from the group comprising halogen, nitro, cyano, hydroxyl, hydroxymethyl, trifluoromethyl and trifluoromethoxy or by straight-chain or branched alkyl or alkoxy each having up to 6 carbon atoms,
or,
represent tetrazolyl, each of which is optionally substituted by methyl or a trityl group, or represent a group of the formula 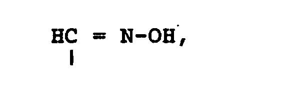 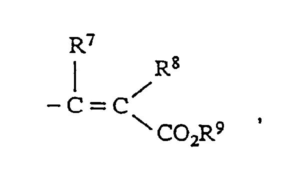 -NR¹⁰R¹¹, -CO-NR¹⁰R¹¹ or -CH₂-OR¹²
in which
R⁷ denotes hydrogen or straight-chain or branched alkyl having up to 6 carbon atoms,
R⁸ denotes hydrogen or straight-chain or branched alkyl having up to 8 carbon atoms, which is optionally substituted by cycloalkyl having 3 to 6 carbon atoms, phenyl or by a 5- to 7-membered saturated or unsaturated heterocycle having up to 3 heteroatoms from the series comprising S, N and O, where the cycles are optionally substituted by hydroxyl, hydroxymethyl or halogen or by straight-chain or branched alkyl or alkoxy each having up to 6 carbon atoms, or denotes cycloalkyl having 3 to 6 carbon atoms or phenyl,
R⁹ denotes hydrogen, straight-chain or branched alkyl having up to 8 carbon atoms or phenyl,
R¹⁰ o and R¹¹ are identical or different and denote hydrogen, cycloalkyl having 3 to 8 carbon atoms or straight-chain or branched alkyl having up to 8 carbon atoms, which is optionally substituted by phenyl,
R¹² denotes straight-chain or branched acyl having up to 6 carbon atoms or benzoyl, or -A-R¹ and R² together represent an alkylene chain having up to 5 carbon atoms,
R⁵ represents hydrogen, halogen, cyano, nitro, trifluoromethyl, hydroxyl, trifluoromethoxy or straight-chain or branched alkyl or alkoxy each having up to 6 carbon atoms,
D represents a radical of the formula 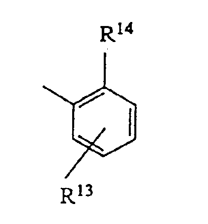 in which
R¹3 3 has the abovementioned meaning of R⁵ and is identical to or different from this, and
R14 denotes a group of the formula -CO-R¹⁵, -CO-NR¹⁶R¹⁷ or -SO₂R¹⁸, in which
R1⁵ denotes hydroxyl or straight-chain or branched alkoxy having up to 6 carbon atoms,
R¹⁶ and R¹⁷ are identical or different and have the abovementioned meaning of R¹⁰ and R¹¹, or
R¹⁶ denotes hydrogen
and
R¹⁷ denotes the group -SO₂R¹⁸,
R¹⁸ denotes hydroxyl, straight-chain or branched alkoxy or alkyl each having up to 6 carbon atoms, amino or (C₁-C₆)-mono- or -dialkylamino or phenyl which can optionally be substituted up to 2 times by identical or different substituents from the group comprising halogen, trifluoromethyl and straight-chain or branched alkyl having up to 4 carbon atoms,
or
R¹⁴ denotes a radical of the formula 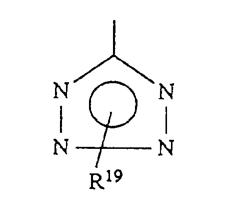 in which
R¹⁹ denotes hydrogen or straight-chain or branched alkyl having up to 8 carbon atoms, which is optionally substituted by straight-chain or branched acyl having up to 6 carbon atoms or denotes the triphenylmethyl group,
and their salts, characterised in that pyridones of the general formula (II) 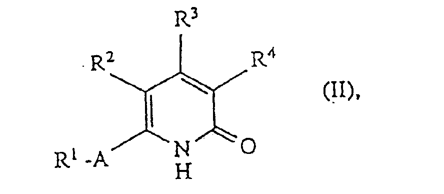 in which
R¹, R2, R4 and A have the given meaning,
are reacted with compounds of the general formula (III) 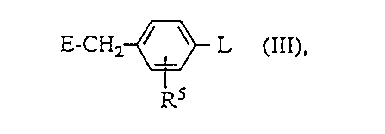 in which
represents halogen, preferably bromine, and
L represents a radical of the formula 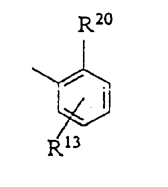 in which
R13 3 has the abovementioned meaning and
R²0 denotes C₁-C₄-alkoxycarbonyl or a radical of the formula 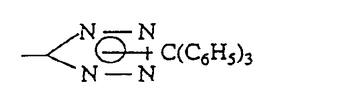 in inert solvents, in the presence of a base and if appropriate with the addition of a catalyst, and then in the case of the tetrazole radical the triphenylmethyl group is removed with acids in organic solvents and/or water according to customary conditions,
and if appropriate in the case of the carbonyl radicals mentioned under the substituents R¹⁴ and/or R²⁰, derivatised after hydrolysis of the respective esters, for example, by amidation or sulphonamidation according to customary methods,
and, if appropriate, the substituents R², R3, R⁴, R⁵, R¹3 and R¹9 are also varied according to the known methods.

2. Process according to Claim 1 for the preparation of substituted biphenylpyridones of the formula (I), in which
R¹ represents straight-chain or branched alkyl, each having up to 8 carbon atoms, which is optionally substituted by cyclopropyl, cyclopentyl, cyclohexyl or hydroxyl or by straight-chain or branched alkoxy having up to 4 carbon atoms, or represents cyclopropyl, cyclopentyl, cyclohexyl, fluorine, chlorine, bromine or iodine,
A represents an oxygen or sulphur atom, or the NH group,
R2, R3 and R4 are identical or different and represent hydrogen, nitro, cyano, formyl, fluorine, chlorine, bromine or iodine, or
represent straight-chain or branched alkyl, alkenyl, alkinyl, alkoxy, or alkylthio each having up to 6 carbon atoms, each of which is optionally substituted by hydroxyl, cyano, fluorine, chlorine, bromine, carboxyl, straight-chain or branched alkoxy, acyl or alkoxycarbonyl each having up to 4 carbon atoms, or by benzyl, phenyl, phenoxy, benzoyl or thienyl, where the cycles can in turn be substituted bytrifluoromethoxy, trifluoromethyl, hydroxymethyl, fluorine, chlorine, bromine, iodine or by straight-chain or branched alkyl or alkoxy each having up to 6 carbon atoms, or
represent straight-chain or branched acyl or alkoxycarbonyl each having up to 6 carbon atoms, phenoxycarbonyl, benzyloxycarbonyl or carboxyl or represent cyclopentenyl, cyclopropyl, cyclopentyl, cyclohexyl, thienyl, furyl, phenyl, phenoxy or phenylthio, each of which is optionally substituted up to 2 times by identical or different substituents from the group comprising fluorine , chlorine, bromine, iodine, trifluoromethyl, trifluoromethoxy and hydroxymethyl or by straight-chain or branched alkyl or alkoxy each having up to 4 carbon atoms,
or
represent tetrazolyl, each of which is optionally substituted by methyl or a trityl group, or represent a group of the formula 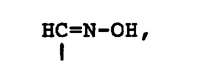 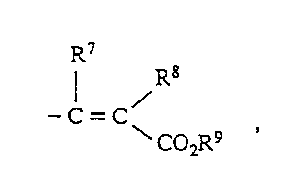 -NR¹⁰R¹¹, -CO-NR¹⁰-R¹¹ or -CH₂-OR¹²,
in which
R⁷ denotes hydrogen or straight-chain or branched alkyl having up to 4 carbon atoms,
R8 denotes hydrogen or straight-chain or branched alkyl having up to 6 carbon atoms, which is optionally substituted by cyclopropyl, cyclohexyl, thienyl or phenyl, where the cycles are optionally substituted by hydroxyl, hydroxymethyl or straight-chain or branched alkyl or alkoxy each having up to 4 carbon atoms,
R9 denotes hydrogen, straight-chain or branched alkyl having up to 6 carbon atoms,
R¹⁰ and R¹¹ are identical or different and denote hydrogen, cyclopropyl, cyclopentyl, cyclohexyl or straight-chain or branched alkyl having up to 6 carbon atoms, which is optionally substituted by phenyl,
R¹² denotes straight-chain or branched acyl having up to 6 carbon atoms or benzoyl, or -A-R¹ and R² together represent an alkylene chain having up to 5 carbon atoms,
R⁵ represents hydrogen, fluorine, chlorine, bromine, hydroxyl, trifluoromethyl, trifluoromethoxy or straight-chain or branched alkyl or alkoxy each having up to 4 carbon atoms,
D represents a radical of the formula 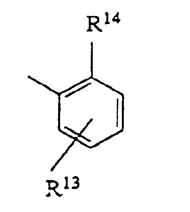 in which
R¹3 has the abovementioned meaning of R⁵ and is identical to or different from this and
R¹⁴ denotes a group of the formula -CO-R¹⁵, -CO-NR¹⁶R¹⁷ or -S0₂R¹⁸, in which
R¹⁵ denotes hydroxyl or straight-chain or branched alkoxy having up to 4 carbon atoms,
R¹ and R¹⁷ are identical or different and have the abovementioned meaning of R¹⁰ and R¹¹ or
R¹⁶ denotes hydrogen and
R¹⁷ denotes the group -SO₂R¹⁸,
R¹⁸ denotes straight-chain or branched alkyl having up to 4 carbon atoms or p-tolyl, or
R¹⁴ 4 denotes a radical of the formula 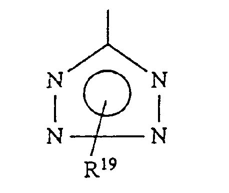 in which
R¹⁹ denotes hydrogen or straight-chain or branched alkyl having up to 6 carbon atoms, which is optionally substituted by straight-chain or branched acyl having up to 4 carbon atoms or denotes the triphenylmethyl group,
and their salts.

3. Process for the preparation of substituted biphenylpyridones of the formula 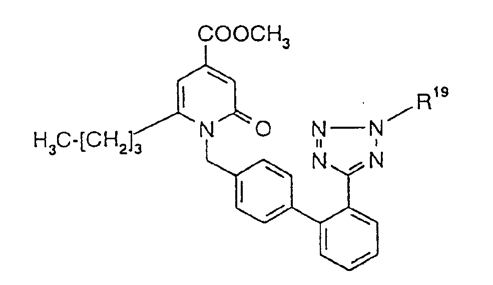 in which
R19 9 represents hydrogen or represents the triphenylmethyl radical, and their salts, characterised in that pyridones of the formula (II) 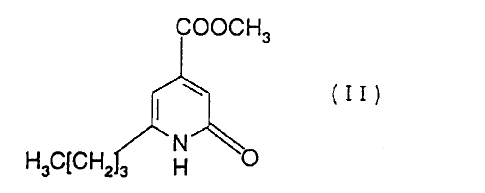 are reacted with compounds of the general formula (III) 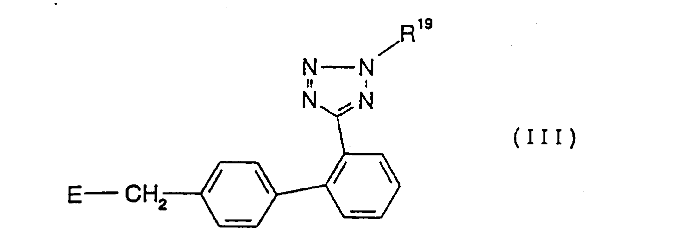 in which
E represents halogen, preferably bromine, in inert solvents, in the presence of a base and if appropriate with the addition of a catalyst,
and then in the case of the tetrazole radical the triphenylmethyl group is removed with acids in organic solvents and/or water according to customary conditions.

4. Process according to Claim 3 for the preparation of 6-butyl-4-methoxycarbonyl-2-oxo-1-[2'-tetrazol-yl-biphenyl-4-yl)methyl]-1,2-dihydropyridine and its salts.

5. Process according to Claims 1 to 4, characterised in that the reaction is carried out in a temperature range from - 100°C to +100°C.

## Revendications (Revendications pour l'(les) Etat(s) contractant(s) suivant(s) : AT, BE, CH, DE, DK, FR, GB, GR, IE, IT, LI, LU, MC, NL, PT, SE)

1. Biphénylpyridones substituées de formule générale 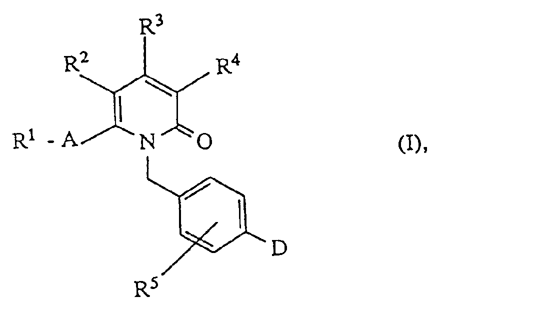 dans laquelle
R¹ est un groupe alkyle linéaire ou ramifié ayant jusqu'à 10 atomes de carbone, qui est substitué, le cas échéant par un reste cycloalkyle ayant 3 à 6 atomes de carbone, un reste hydroxy ou un reste alkoxy linéaire ou ramifié ayant jusqu'à 6 atomes de carbone, ou représente un groupe cycloalkyle ayant 3 à 6 atomes de carbone ou un halogène,
A est un atome d'oxygène ou de soufre ou représente le groupe de formule -NR⁶ dans laquelle
R6 est de l'hydrogène ou un reste radical alkyle linéaire ou ramifié ayant jusqu'à 6 atomes de carbone, ou
R6 forme conjointement avec R¹, y compris l'atome d'azote, un hétérocycle pentagonal ou hexagonal saturé ou non saturé, R², R3 et R⁴ sont identiques ou différents et représentent, de l'hydrogène, un groupe nitro, cyano, formyle ou un halogène, ou bien
un groupe alkyle, alcényle, alcynyle, alkoxy ou alkylthio linéaire ou ramifié ayant dans chaque cas jusqu'à 8 atomes de carbone, ces groupes étant substitués, le cas échéant, jusqu'à 2 fois identiques ou différentes par un reste hydroxy, cyano, un halogène, un reste carboxy, un reste alkoxy, acyle ou alkoxycarbonyle linéaire ou ramifié ayant chacun jusqu'à 6 atomes de carbone, ou par un reste benzyle, phényle, phénoxy, benzoyle ou par un hétérocycle pentagonal à heptagonal saturé ou non saturé ayant jusqu'à 3 hétéro-atomes, les cycles pouvant être substitués quant à eux jusqu'à 2 fois identiques ou différentes par un reste trifluorométhyle, trifluorométhoxy, halogéno, nitro, cyano, hydroxy, hydroxyméthyle ou par un reste alkyle ou alkoxy linéaire ou ramifié ayant chacun jusqu'à 6 atomes de carbone, ou bien
un groupe acyle ou alkoxycarbonyle linéaire ou ramifié ayant chacun jusqu'à 8 atomes de carbone, un groupe phénoxycarbonyle, benzyloxycarbonyle ou carboxy, ou bien
un groupe cycloalkyle ou cycloalcényle ayant 3 à 8 atomes de carbone, un hétérocycle pentagonal à heptagonal non saturé ayant jusqu'à 3 hétéroatomes de la série S, N ou 0,
un groupe phényle, phénoxy ou phénylthio pouvant être substitué chacun, le cas échéant, jusqu'à 3 fois identiques ou différentes par un halogène, un reste nitro, cyano, hydroxy, hydroxyméthyle, trifluorométhyle, trifluorométhoxy ou par un reste alkyle ou alkoxy linéaire ou ramifié ayant chacun jusqu'à 6 atomes de carbone, ou bien
des groupes tétrazolyle qui sont substitués, le cas échéant par un groupe méthyle ou un groupe trityle, ou bien
un groupe de formule 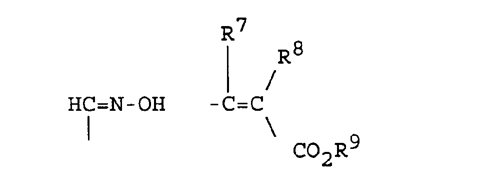 -NR¹⁰R¹¹, -CO-NR¹⁰R¹¹ ou -CH₂-OR¹² dans laquelle
R7 est de l'hydrogène ou un groupe alkyle linéaire ou ramifié ayant jusqu'à 6 atomes de carbone,
R8 est de l'hydrogène ou un groupe alkyle linéaire ou ramifié ayant jusqu'à 8 atomes de carbone, qui est substitué, le cas échéant par un reste cycloalkyle ayant 3 à 6 atomes de carbone, un reste phényle ou un hétérocycle pentagonal à heptagonal saturé ou non saturé ayant jusqu'à 3 hétéro-atomes de la série S, N ou 0, les cycles étant substitués, le cas échéant par un reste hydroxy, hydroxyméthyle, halogéno ou par un reste alkyle ou un reste alkoxy linéaire ou ramifié ayant chacun jusqu'à 6 atomes de carbone, ou un groupe cycloalkyle de 3 à 6 atomes de carbone ou un groupe phényle,
R9 est de l'hydrogène, un groupe alkyle linéaire ou ramifié ayant jusqu'à 8 atomes de carbone ou un groupe phényle,
R¹⁰ et R¹¹ sont identiques ou différents et représentent de l'hydrogène, un groupe cycloalkyle de 3 à 8 atomes de carbone ou un groupe alkyle linéaire ou ramifié ayant jusqu'à 8 atomes de carbone, qui est substitué, le cas échéant par un reste phényle,
R¹² est un groupe acyle linéaire ou ramifié ayant jusqu'à 6 atomes de carbone ou un groupe benzoyle, ou bien -A-R¹ et R² forment ensemble une chaîne alkylénique ayant jusqu'à 5 atomes de carbone,
R⁵ est de l'hydrogène, un halogène, un groupe cyano, nitro, trifluorométhyle, hydroxy, trifluorométhoxy ou un groupe alkyle ou alkoxy linéaire ou ramifié ayant chacun jusqu'à 6 atomes de carbone,
D est un reste de formule 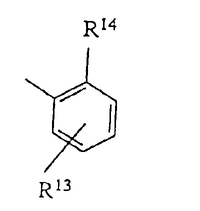 dans laquelle
R¹³ a la définition indiquée ci-dessus pour R⁵
et est identique à ce dernier ou en est différent, et
R¹⁴ est un groupe de formule -CO-R¹⁵, -CO-NR¹⁶R¹⁷ ou S0₂R¹⁸, dans lequel
R¹⁵ 5 est un radical hydroxy ou un radical alkoxy linéaire ou ramifié ayant jusqu'à 6 atomes de carbone,
R¹⁶ et R1⁷ sont identiques ou différents et ont la définition indiquée ci-dessus pour R¹⁰ et R¹¹, ou bien
R¹⁶ est de l'hydrogène et
R¹⁷ est le groupe -S0₂R¹⁸,
R¹⁸ est un radical hydroxy, un radical alkoxy ou un radical alkyle linéaire ou ramifié ayant chacun jusqu'à 6 atomes de carbone, un radical amino ou mono- ou di(alkyle en Ci à C₆)amino ou phényle, qui peut être substitué, le cas échéant jusqu'à 2 fois identiques ou différentes par un halogène, un reste trifluorométhyle ou un reste alkyle linéaire ou ramifié ayant jusqu'à 4 atomes de carbone,
ou bien
R14 est un reste de formule 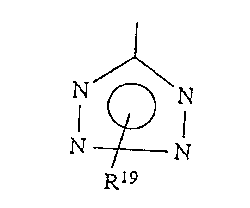 dans laquelle
R19 ⁹ est de l'hydrogène ou un radical alkyle linéaire ou ramifié ayant jusqu'à 8 atomes de carbone, qui est substitué, le cas échéant par un radical acyle linéaire ou ramifié ayant jusqu'à 6 atomes de carbone, ou le groupe triphénylméthyle, et leurs sels.

2. Biphénylpyridones substituées suivant la revendication 1, dans lesquelles
R¹ est un groupe alkyle linéaire ou ramifié ayant jusqu'à 8 atomes de carbone, qui est substitué, le cas échéant par un radical cyclopropyle, cyclopentyle, cyclohexyle, hydroxy ou par un radical alkoxy linéaire ou ramifié ayant jusqu'à 4 atomes de carbone, ou bien
un groupe cyclopropyle, cyclopentyle, cyclohexyle, fluor, du chlore, du brome ou de l'iode,
A représente un atome d'oxygène ou de soufre ou le groupe NH,
R², R3 et R⁴ sont identiques ou différents et représentent
de l'hydrogène un groupe nitro, cyano, formyle, du fluor, du chlore, du brome ou de l'iode, ou bien un groupe alkyle, alcényle, alcynyle, alkoxy ou alkylthio linéaire ou ramifié ayant dans chaque cas jusqu'à 6 atomes de carbone, ces groupes étant substitués, le cas échéant, par un radical hydroxy, cyano, fluoro, chloro, bromo, carboxy, un radical alkoxy, acyle ou alkoxycarbonyle linéaire ou ramifié ayant dans chaque cas jusqu'à 4 atomes de carbone, ou par un radical benzyle, phényle, phénoxy, benzoyle ou thiényle, les cycles pouvant être substitués quant à eux par un radical trifluorométhoxy, trifluorométhyle, hydroxyméthyle, fluoro, chloro, bromo, iodo ou par un radical alkyle ou un radical alkoxy linéaire ou ramifié ayant chacun jusqu'à 6 atomes de carbone, ou bien
un groupe acyle ou alkoxycarbonyle linéaire ou ramifié ayant chacun jusqu'à 6 atomes de carbone, phénoxycarbonyle, benzyloxycarbonyle ou carboxy, ou bien
un groupe cyclopentényle, cyclopropyle, cyclopentyle, cyclohexyle, thiényle, furyle, phényle, phénoxy ou phénylthio, ces groupes pouvant être substitués, le cas échéant, jusqu'à 2 fois identiques ou différentes par du fluor, du chlore, du brome, de l'iode, un radical trifluorométhyle, trifluorométhoxy, hydroxyméthyle ou par un radical alkyle ou un radical alkoxy linéaire ou ramifié ayant chacun jusqu'à 4 atomes de carbone, ou bien
des groupes tétrazolyle qui sont éventuellement substitués par un groupe méthyle ou un groupe trityle, ou bien
un groupe de formule 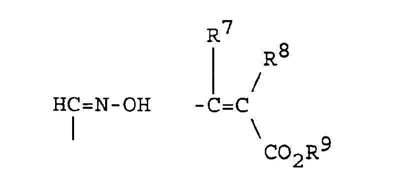
-NR¹⁰R¹¹, -CO-NR¹⁰R¹¹ ou -CH₂-OR¹²
dans laquelle
R⁷ est de l'hydrogène ou un radical alkyle linéaire ou ramifié ayant jusqu'à 4 atomes de carbone,
R8 est de l'hydrogène ou un radical alkyle linéaire ou ramifié ayant jusqu'à 6 atomes de carbone, qui est substitué, le cas échéant par un reste cyclopropyle, cyclohexyle, thiényle ou phényle, les cycles pouvant être substitués, le cas échéant, par un reste hydroxy, hydroxyméthyle ou un reste alkyle ou alkoxy linéaire ou ramifié ayant dans chaque cas jusqu'à 4 atomes de carbone,
R⁹ est de l'hydrogène, un groupe alkyle linéaire ou ramifié ayant jusqu'à 6 atomes de carbone, R¹⁰ et R¹¹ sont identiques ou différents et représentent de l'hydrogène, un reste cyclopropyle, cyclopentyle, cyclohexyle ou un reste alkyle linéaire ou ramifié ayant jusqu'à 6 atomes de carbone, qui est substitué, le cas échéant par un radical phényle,
R¹² est un reste acyle linéaire ou ramifié ayant jusqu'à 6 atomes de carbone ou un reste benzoyle,
ou bien -A-R¹ et R² forment ensemble une chaîne alkylénique ayant jusqu'à 5 atomes de carbone,
R⁵ est de l'hydrogène, du fluor, du chlore, du brome, un reste hydroxy, trifluorométhyle, trifluorométhoxy ou un reste alkyle ou alkoxy linéaire ou ramifié ayant dans chaque cas jusqu'à 4 atomes de carbone,
D est un reste de formule 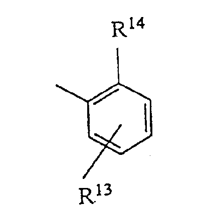 dans laquelle
R¹³ a la définition indiquée ci-dessus pour R⁵ et est identique à ce dernier ou en est différent, et
R¹⁴ est un groupe de formule -CO-R¹⁵, -CO-NR¹⁶R¹⁷ ou -S0₂R¹⁸, dans lequel
R¹⁵ est un radical hydroxy ou un radical alkoxy linéaire ou ramifié ayant jusqu'à 4 atomes de carbone,
R¹⁶ et R17 sont identiques ou différents et ont la définition indiquée ci-dessus pour R¹⁰ et R¹¹, ou bien
R¹⁶ est de l'hydrogène et
R¹⁷ est le groupe -S0₂R¹⁸,
R¹⁸ est un reste alkyle linéaire ou ramifié ayant jusqu'à 4 atomes de carbone ou un reste p-tolyle, ou bien
R¹⁴ est un reste de formule 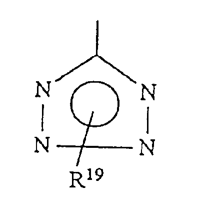 dans laquelle
R¹⁹ est de l'hydrogène ou un groupe alkyle linéaire ou ramifié ayant jusqu'à 6 atomes de carbone, qui est substitué, le cas échéant par un radical acyle linéaire ou ramifié ayant jusqu'à 4 atomes de carbone, ou représente le groupe triphénylméthyle,
et leurs sels.

3. Biphénylpyridones substituées de formule 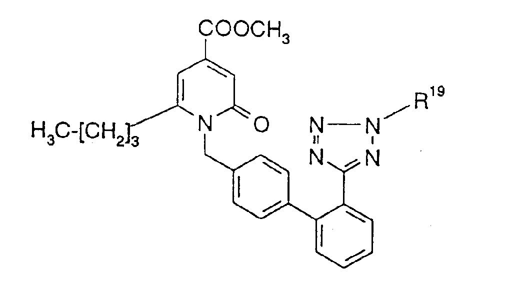 dans laquelle
R¹⁹ représente l'hydrogène ou le reste trifluorométhyle
et leurs sels.

4. La 6-butyl-4-méthoxycarbonyl-2-oxo-1-[(2'-térazolyl-biphényl-4-yl)méthyl]-1,2-dihydropyridine suivant la revendication 3 et ses sels.

5. Biphénylpyridones substituées suivant les revendications 1 à 4, destinées à un traitement thérapeutique.

6. Procédé de production de biphénylpyridones substituées suivant les revendications 1 à 4, caractérisé en ce qu'on fait réagir des pyridones de formule générale (II) 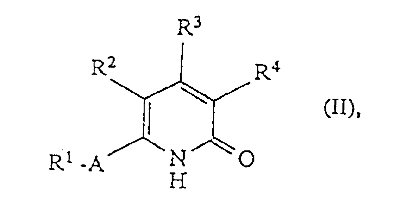 dans laquelle
R¹, R², R⁴ et A ont la définition indiquée ci-dessus, avec des composés de formule générale (III) 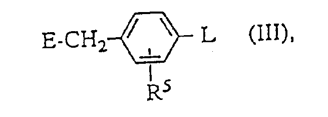 dans laquelle
E représente un halogène, de préférence le brome et
L est un reste de formule 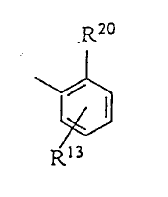
dans laquelle R¹³ a la définition indiquée ci-dessus et
R20 est un groupe (alkox^{y} en Ci à C₄)carbonyle ou
un reste de formule 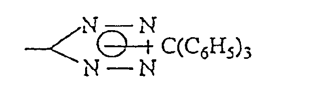
dans des solvants inertes, en présence d'une base et avec addition éventuelle d'un catalyseur,
puis, dans le cas du reste tétrazole, on élimine le groupe triphénylméthyle avec des acides dans des solvants organiques et/ou de l'eau dans des conditions classiques,
et, le cas échéant, dans le cas des restes carbonyle indiqués pour les substituants R¹⁴ et/ou R²⁰, on effectue une dérivatisation par des procédés classiques après saponification des esters correspondants, par exemple par amidation ou sulfamidation,
et on fait aussi varier, le cas échéant, les substituants R², R³, R⁴, R⁵ et R¹³ et R¹⁹ par des procédés connus.

7. Procédé suivant la revendication 6, caractérisé en ce qu'on conduit la réaction dans une plage de températures de -100°C à +100°C.

8. Médicament contenant au moins une biphénylpyridone substituée suivant les revendications 1 à 4.

9. Médicament suivant la revendication 8, destiné au traitement de l'hypertension artérielle et de l'athérosclérose.

10. Procédé de préparation d'un médicament suivant les revendications 8 et 9, caractérisé en ce qu'on fait prendre une forme d'application appropriée aux biphénylpyridones substituées, le cas échéant, à l'aide de substances auxiliaires et de supports appropriés.

11. Utilisation de biphénylpyridones substituées suivant les revendications 1 à 4 pour la préparation de médicaments.

12. Utilisation de biphénylpyridones substituées suivant les revendications 1 à 4 pour la préparation de médicaments destinés au traitement de l'hypertension artérielle et de l'athérosclérose.

## Revendications (Revendications pour l'(les) Etat(s) contractant(s) suivant(s) : ES)

1. Procédé de production de biphénylpyridones substituées de formule (I) 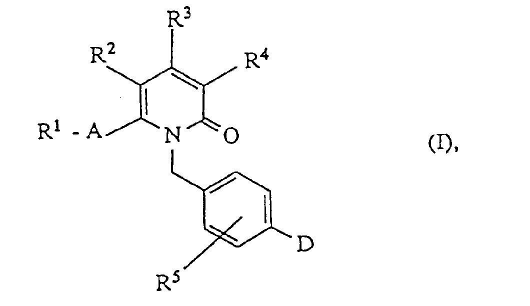 dans laquelle
R¹ est un groupe alkyle linéaire ou ramifié ayant jusqu'à 10 atomes de carbone, qui est substitué, le cas échéant par un reste cycloalkyle ayant 3 à 6 atomes de carbone, un reste hydroxy ou un reste alkoxy linéaire ou ramifié ayant jusqu'à 6 atomes de carbone, ou représente un groupe cycloalkyle ayant 3 à 6 atomes de carbone ou un halogène,
A est un atome d'oxygène ou de soufre ou représente le groupe de formule -NR⁶ dans laquelle
R6 est de l'hydrogène ou un reste radical alkyle linéaire ou ramifié ayant jusqu'à 6 atomes de carbone, ou
R6 forme conjointement avec R¹, y compris l'atome d'azote, un hétérocycle pentagonal ou hexagonal saturé ou non saturé,
R2, R3 et R⁴ sont identiques ou différents et représentent,
de l'hydrogène, un groupe nitro, cyano, formyle ou un halogène, ou bien
un groupe alkyle, alcényle, alcynyle, alkoxy ou alkylthio linéaire ou ramifié ayant dans chaque cas jusqu'à 8 atomes de carbone, ces groupes étant substitués, le cas échéant, jusqu'à 2 fois identiques ou différentes par un reste hydroxy, cyano, un halogène, un reste carboxy, un reste alkoxy, acyle ou alkoxycarbonyle linéaire ou ramifié ayant chacun jusqu'à 6 atomes de carbone, ou par un reste benzyle, phényle, phénoxy, benzoyle ou par un hétérocycle pentagonal à heptagonal saturé ou non saturé ayant jusqu'à 3 hétéro-atomes, les cycles pouvant être substitués quant à eux jusqu'à 2 fois identiques ou différentes par un reste trifluorométhyle, trifluorométhoxy, halogéno, nitro, cyano, hydroxy, hydroxyméthyle ou par un reste alkyle ou alkoxy linéaire ou ramifié ayant chacun jusqu'à 6 atomes de carbone, ou bien
un groupe acyle ou alkoxycarbonyle linéaire ou ramifié ayant chacun jusqu'à 8 atomes de carbone, un groupe phénoxycarbonyle, benzyloxycarbonyle ou carboxy, ou bien
un groupe cycloalkyle ou cycloalcényle ayant 3 à 8 atomes de carbone, un hétérocycle pentagonal à heptagonal non saturé ayant jusqu'à 3 hétéroatomes de la série S, N ou 0,
un groupe phényle, phénoxy ou phénylthio pouvant être substitué chacun, le cas échéant, jusqu'à 3 fois identiques ou différentes par un halogène, un reste nitro, cyano, hydroxy, hydroxyméthyle, trifluorométhyle, trifluorométhoxy ou par un reste alkyle ou alkoxy linéaire ou ramifié ayant chacun jusqu'à 6 atomes de carbone, ou bien
des groupes tétrazolyle qui sont substitués, le cas échéant par un groupe méthyle ou un groupe trityle, ou bien
un groupe de formule 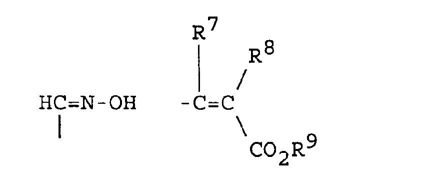 -NR¹⁰R¹¹, -CO-NR¹⁰R¹¹ ou -CH₂-OR¹²
dans laquelle
R7 est de l'hydrogène ou un groupe alkyle linéaire ou ramifié ayant jusqu'à 6 atomes de carbone,
R8 est de l'hydrogène ou un groupe alkyle linéaire ou ramifié ayant jusqu'à 8 atomes de carbone, qui est substitué, le cas échéant par un reste cycloalkyle ayant 3 à 6 atomes de carbone, un reste phényle ou un hétérocycle pentagonal à heptagonal saturé ou non saturé ayant jusqu'à 3 hétéro-atomes de la série S, N ou 0, les cycles étant substitués, le cas échéant par un reste hydroxy, hydroxyméthyle, halogéno ou par un reste alkyle ou un reste alkoxy linéaire ou ramifié ayant chacun jusqu'à 6 atomes de carbone, ou un groupe cycloalkyle de 3 à 6 atomes de carbone ou un groupe phényle,
R9 est de l'hydrogène, un groupe alkyle linéaire ou ramifié ayant jusqu'à 8 atomes de carbone ou un groupe phényle,
R¹⁰ et R¹¹ sont identiques ou différents et représentent de l'hydrogène, un groupe cycloalkyle de 3 à 8 atomes de carbone ou un groupe alkyle linéaire ou ramifié ayant jusqu'à 8 atomes de carbone, qui est substitué, le cas échéant par un reste phényle,
R¹² est un groupe acyle linéaire ou ramifié ayant jusqu'à 6 atomes de carbone ou un groupe benzoyle, ou bien -A-R¹ et R² forment ensemble une chaîne alkylénique ayant jusqu'à 5 atomes de carbone,
R⁵ est de l'hydrogène, un halogène, un groupe cyano, nitro, trifluorométhyle, hydroxy, trifluorométhoxy ou un groupe alkyle ou alkoxy linéaire ou ramifié ayant chacun jusqu'à 6 atomes de carbone,
D est un reste de formule 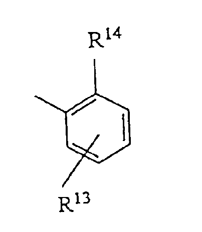 dans laquelle
R¹³ a la définition indiquée ci-dessus pour R⁵ et est identique à ce dernier ou en est différent, et
R¹⁴ est un groupe de formule -CO-R¹⁵, -CO-NR¹⁶R¹⁷ ou SO₂R¹⁸, dans lequel
R¹⁵ 5 est un radical hydroxy ou un radical alkoxy linéaire ou ramifié ayant jusqu'à 6 atomes de carbone,
R¹6 et R¹⁷ sont identiques ou différents et ont la définition indiquée ci-dessus pour R¹⁰ et R¹¹, ou bien
R¹⁶ est de l'hydrogène et
R¹⁷ est le groupe -SO₂R¹⁸,
R¹⁸ est un radical hydroxy, un radical alkoxy ou un radical alkyle linéaire ou ramifié ayant chacun jusqu'à 6 atomes de carbone, un radical amino ou mono- ou di(alkyle en C₁ à C₆)amino ou phényle, qui peut être substitué, le cas échéant jusqu'à 2 fois identiques ou différentes par un halogène, un reste trifluorométhyle ou un reste alkyle linéaire ou ramifié ayant jusqu'à 4 atomes de carbone, ou bien
R¹⁴ est un reste de formule 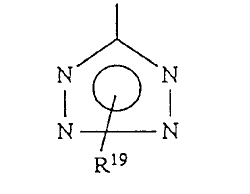 dans laquelle
R¹⁹ est de l'hydrogène ou un radical alkyle linéaire ou ramifié ayant jusqu'à 8 atomes de carbone, qui est substitué, le cas échéant par un radical acyle linéaire ou ramifié ayant jusqu'à 6 atomes de carbone, ou le groupe triphénylméthyle,
et leurs sels,
caractérisé en ce qu'on fait réagir des pyridones de formule générale (II) 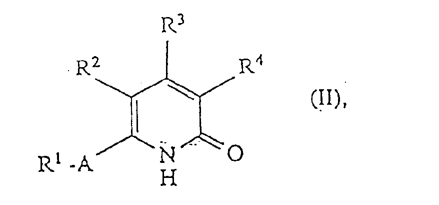 dans laquelle
R¹, R², R⁴ et A ont la définition indiquée ci-dessus, avec des composés de formule générale (III) 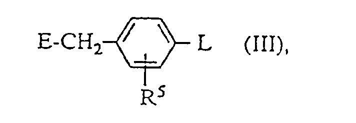 dans laquelle
E représente un halogène, de préférence le brome et
L est un reste de formule 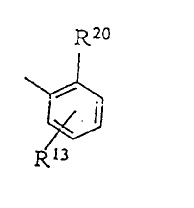 dans laquelle R¹³ a la définition indiquée ci-dessus et
R²⁰ est un groupe (alkoxy en C₁ à C₄)carbonyle ou un reste de formule 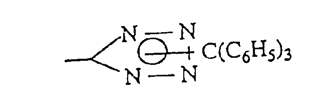
dans des solvants inertes, en présence d'une base et avec addition éventuelle d'un catalyseur,
puis, dans le cas du reste tétrazole, on élimine le groupe triphénylméthyle avec des acides dans des solvants organiques et/ou de l'eau dans des conditions classiques,
et, le cas échéant, dans le cas des restes carbonyle indiqués pour les substituants R¹⁴ et/ou R²⁰, on effectue une dérivatisation par des procédés classiques après saponification des esters correspondants, par exemple par amidation ou sulfamidation,
et on fait aussi varier, le cas échéant, les substituants R², R3, R⁴, R⁵ et R¹3 et R¹⁹ par des procédés connus.

2. Procédé suivant la revendication 1, pour la production de biphénylpyridones substituées de formule (I) dans laquelle
R¹ est un groupe alkyle linéaire ou ramifié ayant jusqu'à 8 atomes de carbone, qui est substitué, le cas échéant par un radical cyclopropyle, cyclopentyle, cyclohexyle, hydroxy ou par un radical alkoxy linéaire ou ramifié ayant jusqu'à 4 atomes de carbone, ou bien
un groupe cyclopropyle, cyclopentyle, cyclohexyle, fluor, du chlore, du brome ou de l'iode,
A représente un atome d'oxygène ou de soufre ou le groupe NH,
R2, R3 et R⁴ sont identiques ou différents et représentent
de l'hydrogène, un groupe nitro, cyano, formyle, du fluor, du chlore, du brome ou de l'iode, ou bien un groupe alkyle, alcényle, alcynyle, alkoxy ou alkylthio linéaire ou ramifié ayant dans chaque cas jusqu'à 6 atomes de carbone, ces groupes étant substitués, le cas échéant, par un radical hydroxy, cyano, fluoro, chloro, bromo, carboxy, un radical alkoxy, acyle ou alkoxycarbonyle linéaire ou ramifié ayant dans chaque cas jusqu'à 4 atomes de carbone, ou par un radical benzyle, phényle, phénoxy, benzoyle ou thiényle, les cycles pouvant être substitués quant à eux par un radical trifluorométhoxy, trifluorométhyle, hydroxyméthyle, fluoro, chloro, bromo, iodo ou par un radical alkyle ou un radical alkoxy linéaire ou ramifié ayant chacun jusqu'à 6 atomes de carbone, ou bien un groupe acyle ou alkoxycarbonyle linéaire ou ramifié ayant chacun jusqu'à 6 atomes de carbone, phénoxycarbonyle, benzyloxycarbonyle ou carboxy, ou bien
un groupe cyclopentényle, cyclopropyle, cyclopentyle, cyclohexyle, thiényle, furyle, phényle, phénoxy ou phénylthio, ces groupes pouvant être substitués, le cas échéant, jusqu'à 2 fois identiques ou différentes par du fluor, du chlore, du brome, de l'iode, un radical trifluorométhyle, trifluorométhoxy, hydroxyméthyle ou par un radical alkyle ou un radical alkoxy linéaire ou ramifié ayant chacun jusqu'à 4 atomes de carbone, ou bien
des groupes tétrazolyle qui sont éventuellement substitués par un groupe méthyle ou un groupe trityle, ou bien
un groupe de formule 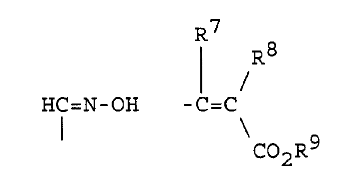 -NR¹⁰R¹¹, -CO-NR¹⁰R¹¹ OU -CH₂-OR¹² dans laquelle
R⁷ est de l'hydrogène ou un radical alkyle linéaire ou ramifié ayant jusqu'à 4 atomes de carbone,
R8 est de l'hydrogène ou un radical alkyle linéaire ou ramifié ayant jusqu'à 6 atomes de carbone, qui est substitué, le cas échéant par un reste cyclopropyle, cyclohexyle, thiényle ou phényle les cycles pouvant être substitués, le cas échéant, par un reste hydroxy, hydroxyméthyle ou un reste alkyle ou alkoxy linéaire ou ramifié ayant dans chaque cas jusqu'à 4 atomes de carbone,
R9 est de l'hydrogène, un groupe alkyle linéaire ou ramifié ayant jusqu'à 6 atomes de carbone,
R¹⁰ o et R¹¹ sont identiques ou différents et représentent de l'hydrogène, un reste cyclopropyle, cyclopentyle, cyclohexyle ou un reste alkyle linéaire ou ramifié ayant jusqu'à 6 atomes de carbone, qui est substitué, le cas échéant par un radical phényle,
R¹² est un reste acyle linéaire ou ramifié ayant jusqu'à 6 atomes de carbone ou un reste benzoyle,
ou bien -A-R¹ et R² forment ensemble une chaîne alkylénique ayant jusqu'à 5 atomes de carbone,
R⁵ est de l'hydrogène, du fluor, du chlore, du brome, un reste hydroxy, trifluorométhyle, trifluorométhoxy ou un reste alkyle ou alkoxy linéaire ou ramifié ayant dans chaque cas jusqu'à 4 atomes de carbone,
D est un reste de formule 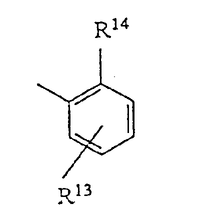 dans laquelle
R¹³ 3 a la définition indiquée ci-dessus pour R⁵ et est identique à ce dernier ou en est différent, et
R¹⁴ est un groupe de formule -CO-R¹⁵, -CO-NR¹⁶R¹⁷ ou -SO₂R¹⁸, dans lequel
R¹⁵ est un radical hydroxy ou un radical alkoxy linéaire ou ramifié ayant jusqu'à 4 atomes de carbone,
R¹⁶ et R¹⁷ sont identiques ou différents et ont la définition indiquée ci-dessus pour R¹⁰ et R¹¹, ou bien
R¹⁶ est de l'hydrogène et
R¹⁷ est le groupe -S0₂R¹⁸,
R¹⁸ est un reste alkyle linéaire ou ramifié ayant jusqu'à 4 atomes de carbone ou un reste p-tolyle, ou bien
R¹⁴ est un reste de formule 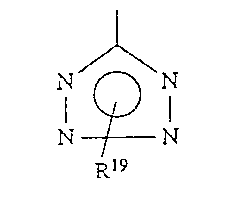 dans laquelle
R¹⁹ est de l'hydrogène ou un groupe alkyle linéaire ou ramifié ayant jusqu'à 6 atomes de carbone, qui est substitué, le cas échéant par un radical acyle linéaire ou ramifié ayant jusqu'à 4 atomes de carbone, ou représente le groupe triphénylméthyle,
et leurs sels.

3. Procédé de production de biphénylpyridones substituées de formule 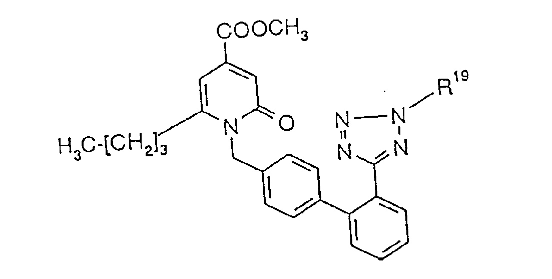 dans laquelle
R¹9 est de l'hydrogène ou le reste triphénylméthyle
et leurs sels,
caractérisé en ce qu'on fait réagir des pyridones de formule (II) 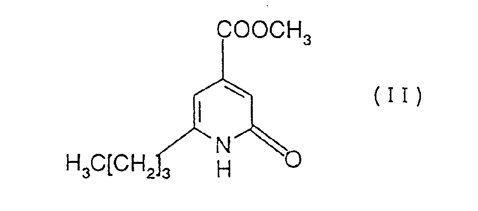 avec des composés de formule générale (III) 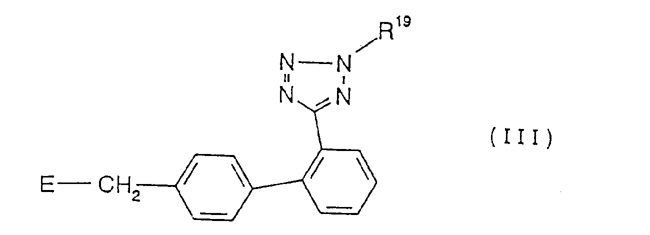 dans laquelle
E représente un halogène, de préférence le brome, et dans des solvants inertes, en présence d'une base et, le cas échéant, avec addition d'un catalyseur,
puis, dans le cas du reste tétrazole, on élimine le groupe triphénylméthyle dans des conditions classiques avec des acides dans des solvants organiques et/ou dans l'eau.

4. Procédé suivant la revendication 3, pour l'obtention de la 6-butyl-4-méthoxycarbonyl-2-oxo-1-[2'-tétrazolyl-biphényl-4-yl)méthyl]-1,2-dihydropyridine et de ses sels.

5. Procédé suivant les revendications 1 à 4, caractérisé en ce qu'on conduit la réaction dans un intervalle de température de -100°C à +100°C.
